# EUROPEAN PATENT SPECIFICATION

(11) **EP 0 514 139 B1**
(45) Date of publication and mention of the grant of the patent: **18.08.1999**
(21) Application number: 92304275.8
(22) Date of filing: 12.05.1992
(51) Int. Cl.: A61B 17/072

(54) **Surgical stapler with spent cartridge sensing and lockout means**
Chirurgisches Klammergerät mit Detektiervorrichtung für verbrauchte Patronen und Sperrvorrichtung
Agrafeuse chirurgicale avec moyens de détection d'une cartouche vide et moyens de blocage

(30) Priority: 14.05.1991 US 699718
(43) Date of publication of application: 19.11.1992
(60) Divisional application: 93120901.9
(73) Proprietor: UNITED STATES SURGICAL CORPORATION, Norwalk, Connecticut 06850 (US)
(72) Inventor: Solyntjes, Alan J., c/o Minnesota Mining and, St. Paul, Minnesota 55133-3427 (US); Eyerly, Robert M., c/o Minnesota Mining and, St. Paul, Minnesota 55133-3427 (US)
(74) Representative: Ritter und Edler von Fischern, Bernhard,Dipl.-Ing.

(56) References cited:
- EP-A- 0 489 436
- GB-A- 2 141 066
- US-A- 4 607 636
- US-A- 4 955 959

## Description

### Technical Field

The present invention relates generally to surgical stapling instruments and more particularly to the type of surgical stapling instruments used for applying linear parallel rows of staggered staples through compressed living tissue.

### Background Art

Surgical stapling instruments used for applying parallel rows of staples through compressed living tissue are well known in the art, and are commonly used for closure of tissue or organs prior to transection, prior to resection, or in anastomoses, and for occlusion of organs in thoracic and abdominal plasty procedures.

One known pneumo-intestinal surgical stapling instrument of this type has been in use for many years, and is currently available under the trade designation "The PI Stapler", catalog # 3960 by minnesota Mining and Manufacturing Company (3M), St. Paul, Minnesota, the use of which stapler is described in a publication entitled "Surgical Stapling, Gastric and Small Bowel Procedures, Volume I". ISBN 0-937433-00-4, Library of Congress Catalog Number 85-082599 available from Minnesota Mining and Manufacturing Company (3M), St. Paul, Minnesota. That stapler and a similar stapler described in Freund et al. PCT Application No. WO 83/02247, published July 7, 1983 are adapted for firing staples into compressed living tissue from a staple filled cartridge or housing. The staplers have anvil and jaw portions, a cartridge holder including a removable cartridge and a handle lever.

"PI" type staplers have a handle lever, a generally C-shaped anvil portion having free and supported ends with an alignment aperture generally adjacent its free end, a cartridge transport member adapted to releasably receive a cartridge or staple housing having an alignment through passage, and an alignment pin movable between an engaged or alignment position with the alignment pin extending through the through passage in the staple housing and into the alignment aperture in the anvil jaw and a release or disengaged position with the alignment pin spaced from the alignment through passage and alignment aperture to afford removal and replacement of a spent staple cartridge.

The anvil and cartridge transport members are each elongate in a longitudinal direction, and the anvil portion has specially shaped anvil surfaces situated in a plane generally perpendicular to the longitudinal direction. When the alignment pin extends through the through passage in the staple housing and into the alignment aperture in the anvil jaw (i.e. when the alignment pin is in the alignment position), the specially shaped anvil surfaces are positioned generally opposite longitudinal slots in the cartridge or staple housing which contain unfired staples. The cartridge transport member/cartridge housing is movable between a closed or clamping position with the cartridge housing and the anvil surfaces in closely spaced relationship, and an open position with the cartridge housing and the anvil surfaces spaced farther from each other than in the closed position.

Typically, a "PI" type stapler is positioned adjacent the tissue to be stapled, the anvil and jaw portions are approximated adjacent the tissue to be stapled, the alignment pin is moved to the alignment position, and the stapler is clamped on the tissue by moving the handle lever in a first movement to cause the cartridge holder to move toward the anvil placing the cartridge housing in a closed "clamping" position where the staples may be fired. Moving the handle lever in a second movement "fires" the stapler (e.g. it ejects the staples from the cartridge).

In some surgical procedures the clamping force results in tissue that is highly compressed to ensure, inter alia, proper hemostasis in the tissues being stapled. The clamping force is present in various degrees in each of the surgical procedures for a "PI" type surgical stapler. Such a clamping force causes tissue trauma in the tissue to be stapled, at least to some degree.

Under a great clamping force, a prior art "PI" type stapler without an engaged alignment pin typically "scissors" or fails by deflection of the anvil portion and the cartridge housing laterally relative to one another (e.g. where the anvil portion deflects laterally with respect to the longitudinal axis of the stapler and/or where the cartridge housing deflects in a lateral direction opposite the deflection direction of the anvil portion).

Moving the alignment pin to the alignment position before firing the stapler or before clamping tissue between the anvil and cartridge housing affords a more precisely controlled formation of the fired staples by aligning the specially shaped surfaces on the anvil portions with the staples in the cartridge housing and by preventing the cartridge housing and the specially shaped anvil surfaces from "scissoring". Moving the alignment pin to the alignment position also prevents tissue from escaping from between the anvil and cartridge housing when the cartridge assembly is clamped on the tissue to be stapled.

The prior art "PI" type staplers encounter problems because it is possible to fire the stapler or clamp tissue between the anvil and cartridge housing without the alignment pin in the alignment position. It is believed that it is difficult to determine whether the alignment pins of the prior art "PI" type staplers are engaged, particularly in the surgical environment. Also, it is possible to inadvertently fail to move the alignment pin to the alignment position. If the alignment pin is not moved to the alignment position before clamping tissue between the anvil and cartridge housing, tissue intended to be stapled may escape from between the anvil and cartridge housing resulting in incomplete anastomoses and other undesirable results. Additionally, if the alignment pin is not moved to the alignment position before the stapler is fired, the staples may be improperly formed.

The prior art "PI" type staplers also encounter problems because it is difficult to determine when they are loaded with a "spent" cartridge or with a cartridge that does not contain staples. on occasion, a spent cartridge may be inadvertently left in a stapler after it has been fired during a surgical procedure where the stapler is used several times for the same patient, or a spent cartridge may be inadvertently loaded into a stapler that is about to be fired in the patient. If a stapler is loaded with a cartridge housing other than a ready-to-fire cartridge housing and the stapler is clamped on tissue to be stapled, the compressive forces created by the stapler subject the tissue to undesirable and unnecessary trauma. The sequence of (1) clamping the stapler with a spent cartridge on tissue, (2) firing the "dud" stapler (3) subsequently rearming, (4) again approximating the stapler adjacent the tissue to be stapled and (5) reclamping the stapler wastes precious time during the surgical procedure.

Additionally, the prior art "PI" type staplers may be used in procedures in which the surgeon uses a scalpel to manually create an incision on a side of closed staples (e.g. the procedure described in the article entitled "Resection of the Lesion" on pages 14 and 15 of the publication entitled "Surgical Stapling, Gastric and Small Bowel Procedures, Volume I", ISBN 0-937433-00-4, Library of Congreas Catalog Number 85-082599 available from Minnesota Mining and Manufacturing Company (3M), St. Paul, Minnesota). A spent stapler cartridge used in such procedures may result in unnecessary blood loss, inadequate hemostasis and tissue trauma for the patient undergoing the procedure.

Surgical stapler cartridge safety devices are described in U.S. Patents No. 4,863,088 and 4,955,959, and U.S. Patent Application 07/629,597. The firing mechanisms in those staplers sequentially close staples into tissue. Since each staple is closed within tissue one after another in succession, the force required to fire such staplers is generally less than the force required to fire a "PI" type stapler where all the staples are generally closed in tissue at the same time.

Unlike the present invention wherein the same part (e.g. a lever or "handle") is operable in both a first movement to approximate and clamp tissue and a second movement to close all the staples within tissue generally simultaneously, the "ILA" type staplers described in U.S. Patents No. 4,863,088 and 4,955,959, and U.S. Patent Application 07/629,597 utilize separate and distinct member to (1) sequentially fire the staples (e.g. a firing tab or button) and (2) clamp onto the tissue (an element of the stapler that does not include the firing tab or button).

U.S. Patent No. 4,527,724 to Chow et al. and assigned to Senmed, Inc., of Cincinnati, Ohio discloses a surgical stapler similar to the surgical stapler sold by Ethicon, Inc., of Somerville, N.J. under the trade name "Proximate". That stapler includes a lockout device which precludes rotation of an adjustment knob to clamp the stapler on tissue unless the alignment and retaining pin is shifted to its operative position. The lockout device, however, is only operative until the user slides the alignment and retaining pin to the operative position. Once the user slides the alignment and retaining pin to the operative position, the lockout device is not designed to be easily reactivated. Thus, to defeat the lockout device, a user need only slide the pin to the operative position and then away from the operative position. The stapler has no feature which retains the pin in the operative position when the stapler is clamped on tissue.

EP-A-489436 was published after the date of EPO filing of the present application. It discloses a surgical staple which includes a cartridge of staples and a frame, and a resilient locking clip mounted in the frame to block the approximation of the cartridge and an anvil once the stapler has been fired, by engagement with a recess in the frame.

### Disclosure of the Invention

The present invention is defined in claim 1 below. It will be appreciated that the locking device of the invention is in the frame whereas in EP-A-489436 it is in the cartridge. The invention provides a surgical stapler comprising an anvil frame elongate in a longitudinal direction and including anvil surfaces in a plane generally perpendicular to the longitudinal direction, surfaces defining an alignment aperture opening onto the anvil surfaces, and a cartridge assembly movable relative to the anvil frame between an open position with the cartridge assembly spaced from the anvil surfaces and a closed position with the cartridge assembly and the anvil surfaces in closely spaced relationship. The cartridge assembly comprises a cartridge housing containing a plurality of staples disposed in rows positioned in opposition to the anvil surfaces.

The surgical stapler also includes a longitudinally extending alignment pin mounted on the stapler for movement between an alignment position with the alignment pin extending into the alignment aperture, and a release position with the alignment pin spaced from the alignment aperture.

By including a mechanism for engaging the cartridge assembly and anvil for continuously preventing the cartridge assembly from moving from the open to the closed position whenever the alignment pin is in a position other than the alignment position, and for preventing the alignment pin from moving from the alignment position toward the release position when the cartridge assembly is in the closed position, the present invention provides a stapler which (1) contributes to the proper formation of staples, (2) promotes proper hemostasis in the tissue to be stapled, (3) prevents "scissoring" of the anvil and cartridge portions of the stapler, (4) prevents tissue from escaping from between the anvil and cartridge housing when the cartridge assembly is clamped on the tissue to be stapled, and (5) reduces the likelihood of a weak joint or an absence of blood flow in the joint and tissue.

The stapler according to the invention includes a mechanism for firing the stapler by engaging and closing the staples in tissue between the cartridge housing and the anvil surfaces, and a means preventing the cartridge assembly from moving from the open to the closed position unless the stapler is loaded with a ready-to-fire cartridge housing. The means for preventing the cartridge assembly from moving from the open to the closed position (1) prevents approximation and clamping of living tissue between anvil and cartridge components of the stapler when the stapler is loaded with a spent stapler cartridge, (2) prevents the user from attempting to refire the stapler, and (3) provides a stapler that reduces the chances of unnecessary tissue trauma, blood loss, inadequate hemostasis, and squandered time during surgery.

According to a preferred embodiment of the stapler of the present invention, there is provided a stapling instrument comprising an anvil frame having proximal and distal ends and a pair of lateral side portions that are each elongate in a longitudinal direction and spaced to define a channel therebetween. The anvil frame has a handle portion generally adjacent the proximal end with first and second ends, and a jaw portion having anvil surfaces generally adjacent the distal end. The jaw portion includes surfaces defining an alignment aperture opening onto the anvil surfaces, and the anvil surfaces are positioned in a plane generally perpendicular to the longitudinal direction.

An elongate manually movable handle or lever part having first and second ends is pivotally mounted at its second end to afford pivotal movement of the lever part relative to the anvil frame between a release position with the first end of the lever part being spaced from the first end of the handle portion and an actuation position with the lever part and the handle portion in closely spaced relationship. A biasing means (such as a coil spring mounted at one end to the anvil frame and at the other end to the lever part) biases the lever part toward the release position. Preferably the biasing means comprises a coil spring mounted at one end to the anvil frame and at the other end to the lever part.

A cartridge assembly having proximal and distal ends is mounted in the channel between the lateral side portions for longitudinal movement relative to the anvil frame. The cartridge assembly comprises a cartridge transporting member having first and second side portions that are each elongate in the longitudinal direction and that are spaced to define a ram channel therebetween. The first and second side portions have surfaces defining a cartridge groove generally adjacent the distal end of the cartridge assembly.

The cartridge groove surfaces are adapted to releasably receive a cartridge housing containing a plurality of staples disposed in rows positioned in opposition to the anvil surfaces, and pusher means, such as a pusher, for pressing the staples within the cartridge housing against the anvil surfaces to engage and close the staples in tissue between the cartridge housing and the anvil surfaces. The pusher has a pair of edges and is positioned proximate the staples for movement between pre-fired and fired positions with the pusher adapted to move distally relative to the cartridge housing when the stapler is fired. The cartridge housing also includes surfaces defining an alignment through passage positioned in opposition to the alignment aperture and whose function will be described later.

A means mounts the cartridge assembly for longitudinal movement relative to the anvil frame between a closed position with the cartridge housing and the anvil surfaces in closely spaced relationship, and an open position with the cartridge housing and the anvil surfaces spaced farther from each other than in the closed position.

An elongate T-bar or "ram" is mounted in the ram channel between the first and second side portions for longitudinal movement relative to the cartridge transporting member and the anvil frame. The ram is adapted to engage and drive the pusher distally to fire the stapler when the cartridge housing the anvil surfaces are in the closed position.

An actuation means is present to initially move the cartridge assembly from the open to the closed position by a first movement of the lever part from the release to the actuation position and for subsequently firing the stapler (i. e. moving the ram distally relative to the cartridge transporting member to cause the pusher to eject the staples from the cartridge housing, to press the staples against the anvil surfaces and to engage and close the staples in tissues between the cartridge housing and the anvil jaw portion) by a second movement of the lever part from the release to the actuation position. Another biasing means (such as a coil spring connected between the anvil frame and the ram) biases the cartridge assembly from the closed to the open position. That biasing means preferably comprises a coil spring connected between the anvil frame and the ram.

The actuation means preferably comprises a toggle joint linkage having an over center pivoting portion and first and second ends with the first end fixed to the anvil frame and with the second end connected to the cartridge transporting member. The over center pivoting portion preferably has surfaces adapted to engage the lever part when the lever part is first moved from the release to the actuation position to move the toggle joint linkage from a retracted position past an in-line or centered position with the toggle joint linkage generally straight, to a slightly inverted (relative to the retracted position) extended position to drive the cartridge assembly from the open to the closed positions. The actuation means also preferably includes means for retaining the cartridge assembly in the closed position against the bias of the coil spring that biases the cartridge assembly from the closed to the open position. Preferably, such a means comprises a stop flange on the over center pivoting portion of the toggle joint linkage that prevents the toggle joint linkage from moving past the extended position. Alternatively such a means may comprise a stop surface located on a handle cover.

The actuation means also preferably includes surfaces defining a cam shoulder surface on the ram, and a pawl having first and second ends and a cam surface generally adjacent the second end of the pawl, and means mounting the pawl on the lever part for movement between a first position with the cam surf ace generally spaced from the cam shoulder surface on the ram and a second position with the cam surface engaged with the cam shoulder surface on the ram to afford firing of the stapler by driving the ram distally relative to the cartridge transporting member. Also preferably, the stapler further includes means, such as a torsion spring, for biasing the pawl toward the second position, and the ram has surfaces which are adapted to retain the pawl in the first position until the cartridge assembly is moved from the open to the closed position.

Additionally, the stapler preferably further includes a release arm having a first end pivotally mounted to the proximal end of the anvil frame and a second engagement end. The release arm has surf aces adapted to engage the pawl and the toggle joint linkage to move the pawl from the second to the first position and to move the toggle joint linkage from the extended toward the retracted position to afford movement of the cartridge assembly from the closed to the open position under the bias of the means for biasing the cartridge assembly from the closed to the open position (e.g. the coil spring).

The stapler according to the present invention also includes a means for sensing whether the stapler is loaded with a fired cartridge housing and for preventing the stapler from being closed or fired when loaded with the fired cartridge.

In a first embodiment of a stapler according to the present invention, the means for preventing the cartridge assembly from moving from the open to the closed position when the stapler is loaded with a spent cartridge housing comprises the anvil frame having surfaces defining a safety aperture opening into the surface of the anvil frame and having a bottom surface, and a locking pin having first and second ends. The locking pin is mounted within the safety aperture for movement between a free travel position with the first end of the locking pin abutting an edge of the pusher to afford a single, reciprocating movement of the cartridge assembly between the open and closed positions, and a blocking position with the first end of the locking pin projecting beyond the safety aperture and into the path of the ram to prevent subsequent movement of the cartridge assembly from the open to the closed position. A biasing means biases the locking pin toward the blocking position. That biasing means preferably comprises a coil spring having a first end connected to the second end of the locking pin and a second end connected to the bottom surface of the safety aperture.

In a second embodiment of stapler according to the present invention, the stapler includes a different means for preventing the cartridge assembly from moving from the open to the closed position when the stapler is loaded with a cartridge housing with the pusher in a fired position comprising a locking notch in the cartridge transport member of the cartridge assembly such that the locking pin engages with the surfaces of the locking notch to prevent movement of the cartridge assembly from the open to the closed position when the locking pin is in the blocking position. In the second embodiment, the locking pin engages the locking notch to prevent movement of the cartridge assembly rather than simply acting as an obstacle to the ram.

In a third embodiment of stapler according to the present invention, the locking notch and/or the locking pin have sloped or ramped camming surfaces to cam the locking pin toward the free travel position when the cartridge assembly initially moves from the open to the closed position.

The three embodiments mentioned above may also include a safety guide member. The safety guide member comprises (1) a sleeve slidably mounted on the anvil frame for longitudinal movement relative thereto and having proximal and distal ends, (2) a longitudinally extending alignment pin mounted on the distal end of the sleeve for movement between an alignment position with the pin passing through the cartridge housing alignment through passage and extending into the alignment aperture and a release position with the alignment pin spaced from the alignment through passage and the alignment aperture, and (3) means for preventing the cartridge assembly from moving from the open to the closed positions unless the alignment pin is in the alignment position, and for preventing the alignment pin from moving from the alignment position toward the release position when the cartridge assembly is in the closed position.

The means for preventing the cartridge assembly from moving from the open to the closed positions unless the alignment pin is in the alignment position, and for preventing the alignment pin from moving from the alignment position toward the release position when the cartridge assembly is in the closed position preferably comprises surfaces defining a safety notch in the anvil frame, cartridge transport member and ram, and a safety gate having cam shoulder surfaces and surfaces defining a hole. The surfaces defining a safety notch in the anvil frame, cartridge transport member and ram are aligned when the cartridge assembly is in the open position to define a continuous safety notch extending laterally across the stapler and are staggered when the cartridge assembly is in the closed position.

The safety gate is mounted adjacent the anvil frame for relative movement thereto between a latched position with the safety gate engaged with the surfaces defining the continuous safety notch to prevent relative movement between the cartridge assembly and the anvil frame, and an unlatched position with the safety gate spaced from the safety notches to afford relative movement of the cartridge assembly and the anvil frame between the open and closed positions.

The safety guide member includes safety gate deactivating guides projecting proximally from the proximal end of the sleeve and through the safety gate hole. The guides are adapted to bias the safety gate toward the latched position when the alignment pin is in a position other than the alignment position and have cam surfaces at a proximal end adapted to engage the cam shoulder surfaces of the safety gate to drive the safety gate from the latched to the unlatched position when the alignment pin is moved to the alignment position.

The means for preventing the alignment pin from moving from the alignment position toward the release position when the cartridge assembly is in the closed position includes the safety gate deactivating guides having return cam surfaces generally opposite the cam surfaces adapted to engage surfaces on the safety gate to drive the safety gate from the unlatched toward the latched position when the alignment pin is moved away from the alignment position, and surfaces on the cartridge transport member and ram adapted to engage the safety gate to prevent the return cam surfaces from driving the safety gate from the unlatched position toward the latched position to thereby prevent the alignment pin from moving from the alignment position toward the release position when the cartridge assembly is in the closed position.

### Brief Description of the Drawing

The present invention will be further described with reference to the accompanying drawing wherein like reference numerals refer to like parts in the several views, and wherein:
Figure 1 is a perspective view of the surgical stapling instrument according to the present invention;
Figures 2 through 5 are enlarged first side views of the surgical instrument of Figure 1 which sequentially illustrate the operation of the stapler wherein:

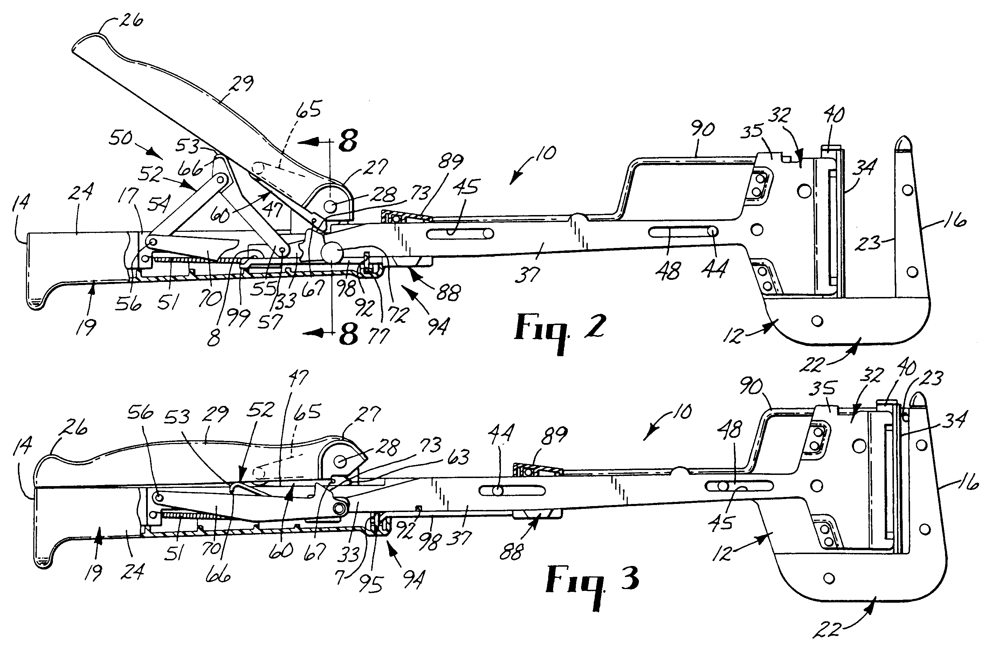

Figure 2 shows the relative positions of the anvil frame and the cartridge assembly in an open position and has portions broken away to show details;

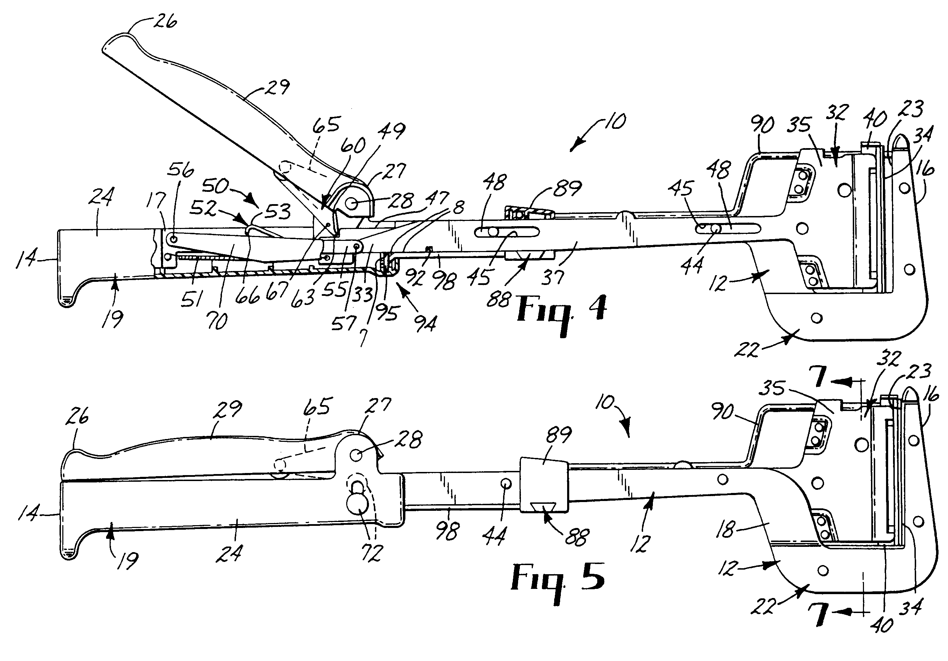

Figure 3 illustrates the positions of the anvil frame and the cartridge assembly just after the cartridge assembly is moved to the closed position and has portions broken away to show details;
Figure 4 shows the positions of the anvil frame and the cartridge assembly just before the stapler is fired and has portions broken away to show details;

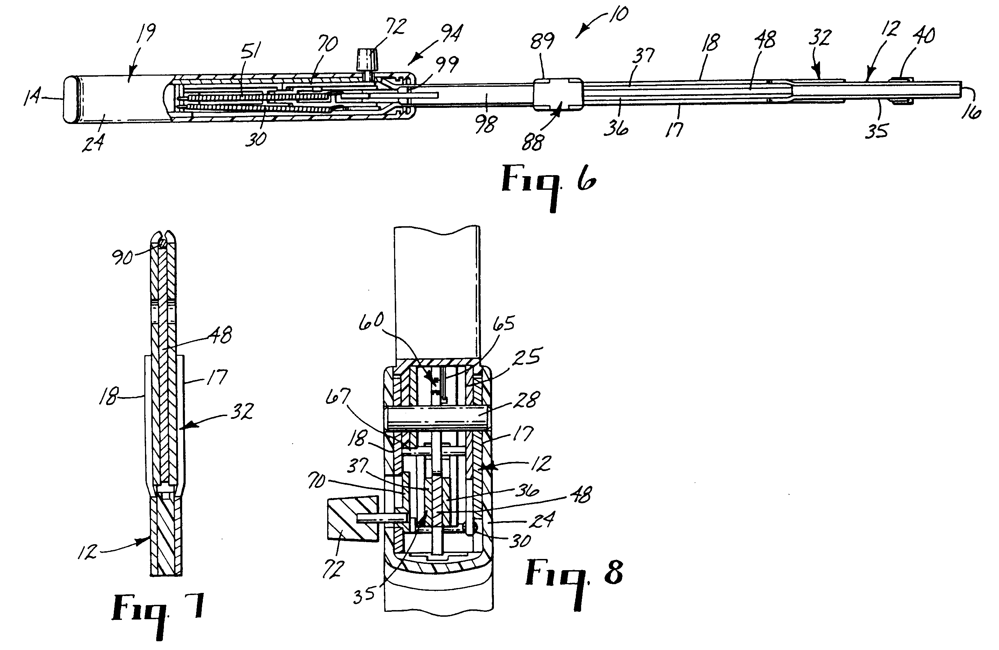

Figure 5 illustrates the stapler just after the stapler is fired;

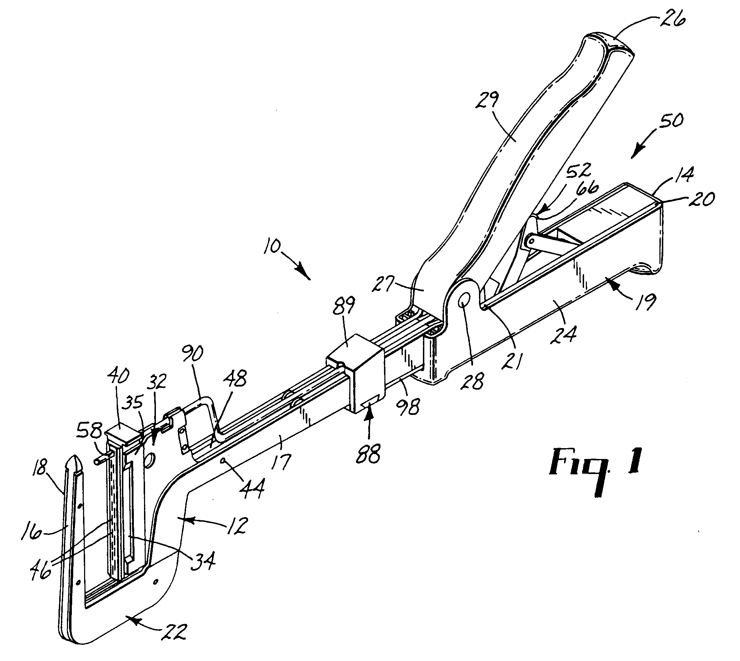

Figure 6 is an enlarged bottom view or the surgical instrument of Figure 1 which has portions broken away to show details;

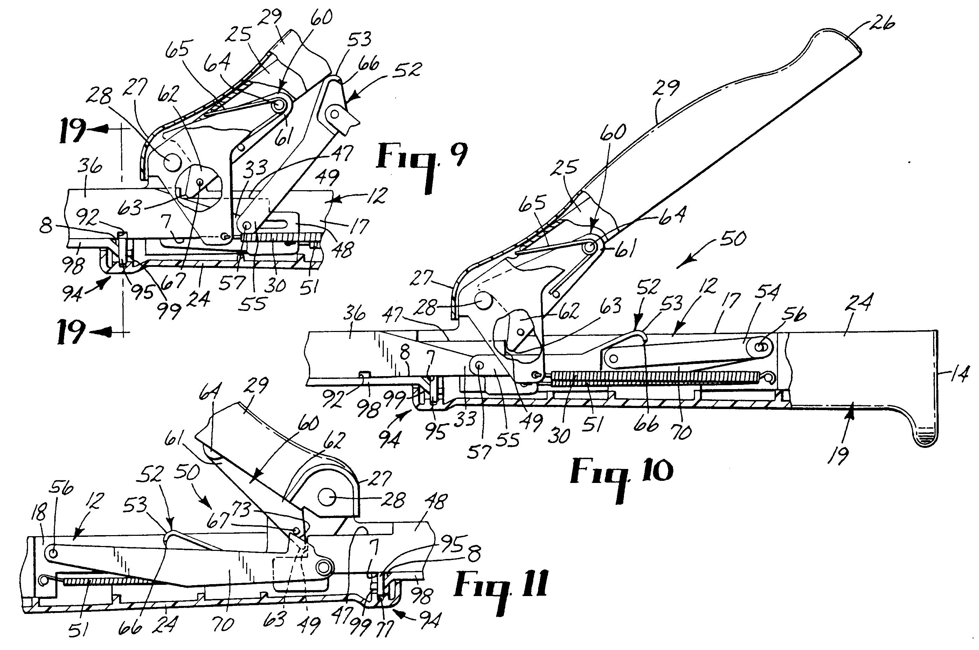

Figure 7 is an enlarged sectional view of the stapler of Figure 1 taken approximately along line 7-7 of Figure 5;
Figure 8 is an enlarged sectional view of the stapler of Figure 1 taken approximately along line 8-8 of Figure 2 and having portions broken away to show details;
Figure 9 is an enlarged second side view of the stapler of Figure 1 having portions broken away to show details of an actuation mechanism;
Figure 10 is an enlarged second side view of the stapler of Figure 1 having portions broken away to show details of an actuation mechanism and which illustrates the proximal end of the stapler;

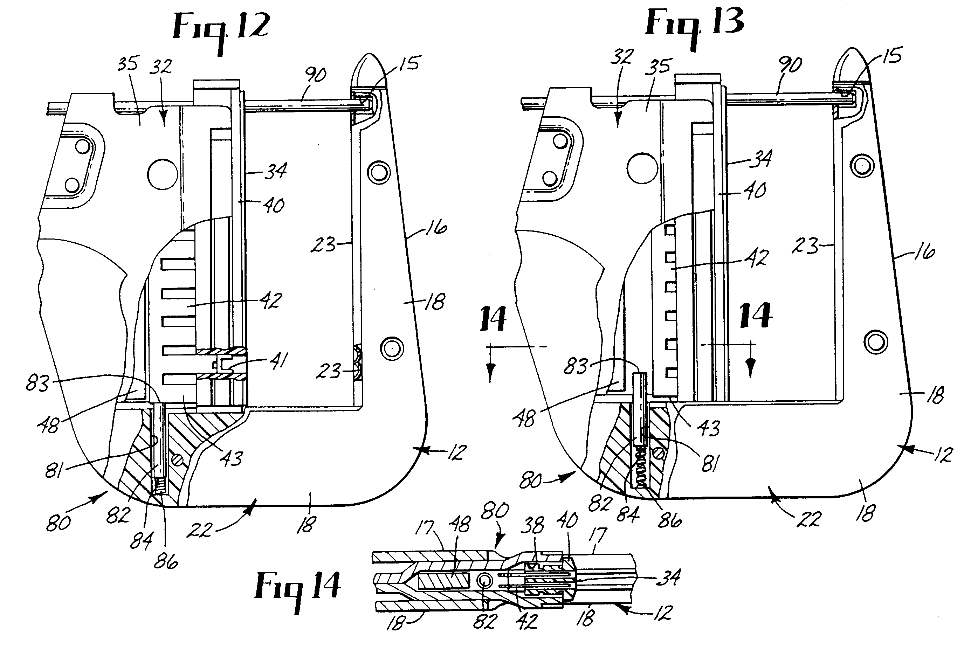

Figure 11 is an enlarged first side view of the stapler of Figure 1 having portions broken away to show details or an actuation mechanism;
Figure 12 is an enlarged first side view or the distal end of the stapler of Figure 1 illustrating a pusher in a pre-fired position;
Figure 13 is an enlarged first side view of the distal end of the stapler of Figure 1 illustrating a pusher in a fired position;
Figure 14 is an enlarged sectional view of the stapler of Figure 13 taken approximately along line 14-14 of Figure 13 and having portions broken away to show details;

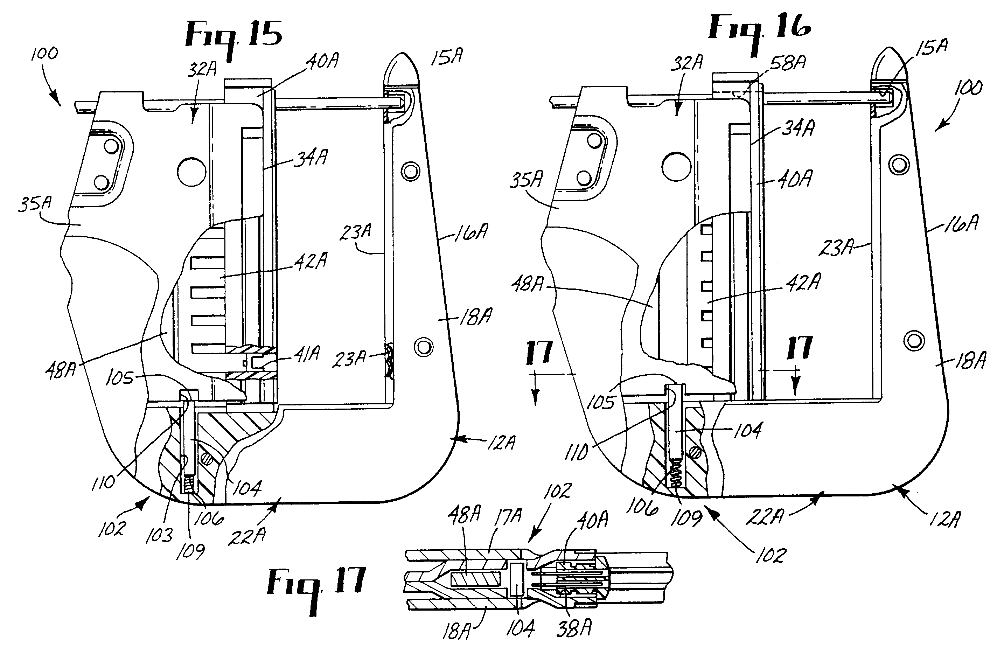

Figure 15 is an enlarged first side view of a distal end of a second embodiment of surgical stapler according to the present invention whose proximal end is generally identical to the proximal end of the stapler of Figure 1, and which illustrates a pusher in a pre-fired position;
Figure 16 is an enlarged first side view of the distal end of the stapler of Figure 15 illustrating a pusher in a fired position;

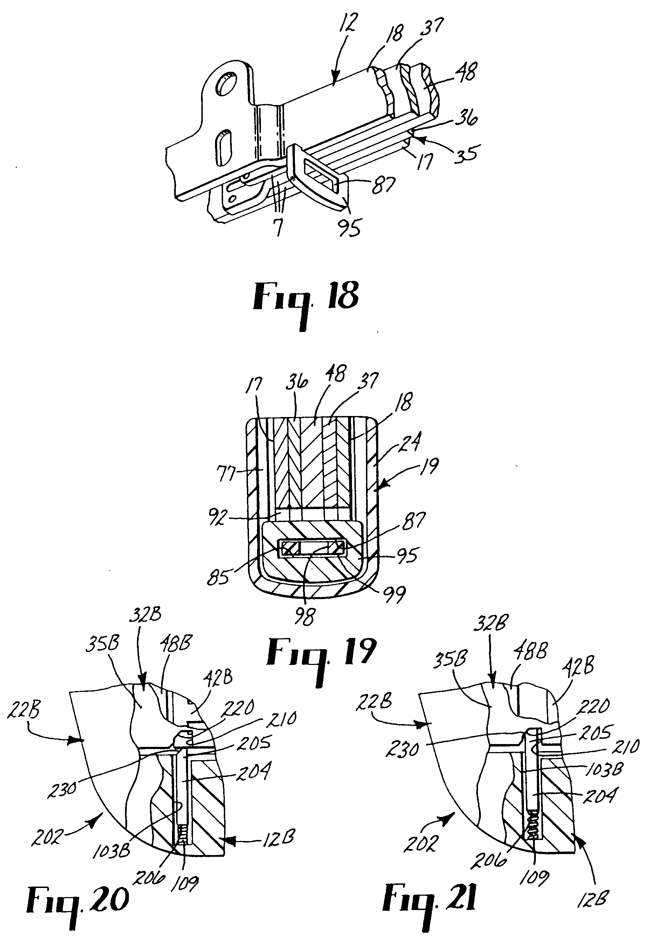

Figure 17 is an enlarged sectional view of the stapler of Figure 15 taken approximately along line 17-17 of Figure 16 and having portions broken away to show details;
Figure 18 is an enlarged perspective view of the stapler of Figure 1 having portions broken away to show detail and showing a safety gate in a latched position;
Figure 19 is a sectional view of the stapler of Figure 1 taken approximately along line 19-19 of Figure 9;
Figure 20 is an enlarged first side view of a distal end of a third embodiment of surgical stapler according to the present invention whose proximal end is generally identical to the proximal end of the stapler of Figure 1, with portions broken away to shown details of a ramped pin and locking notch, illustrating a pusher in a pre-fired position; and
Figure 21 is an enlarged first side view of the distal end of the stapler of Figure 20 illustrating a pusher in a fired position.

### Detailed Description

Referring now to Figures 1 through 14 of the drawing, there is shown a first embodiment of surgical stapling instrument according to the present invention, generally designated by the reference numeral 10.

Generally the surgical stapling instrument 10 comprises an anvil frame 12 having proximal 14 and distal 16 ends and a pair of lateral side portions 17 and 18 that are each elongate in a longitudinal direction and spaced to define a channel therebetween. The anvil frame 12 has a handle portion 19 generally adjacent the proximal end 14 with first 20 and second 21 ends, and a jaw portion 22 having anvil surfaces 23 generally adjacent the distal end 16. The anvil surfaces 23 are specially shaped and are positioned in a plane generally perpendicular to the longitudinal direction. The jaw portion 22 includes surfaces defining an alignment aperture 15 opening onto the anvil surfaces 23, the function of which will be described later.

A handle housing 24 is attached to the anvil frame 12 and may be constructed from any suitable material such as but not limited to a polymeric material such as nylon, polypropylene, high density polyethylene, acrylonitrile butadiene styrene (ABS), polyetherimide, polystyrene, acetal or polycarbonate.

An elongate manually movable handle or lever part 25 (see Figure 9) having first 26 and second 27 ends is pivotally mounted at its second end 27 to the anvil frame 12 by means such as a pin 28. A handle cover 29 is attached to the lever part 25 and may be constructed from any suitable material such as but not limited to a polymeric material similar to the material used to construct the handle housing 24. The handle cover 29 and the handle housing 24 are shaped to afford convenient, efficient manual grasping of the stapler 10 and may be snap fit to the stapler 10 (e.g. the lever part 25 and anvil frame 12 respectively).

The lever part 25 is connected to the anvil frame 12 at a position generally adjacent the second end 21 of the handle portion 19. The pin 28 mounts the handle part 25 and handle cover 29 to the anvil frame 12 to afford pivotal movement of the lever part 25 relative to the anvil frame 12 between a release position (Figures 1, 2 and 4) with the first end 26 of the lover part 25 spaced from the first end 14 of the handle portion 19 and an actuation position (Figures 3 and 5) with the lever part 25 and the handle portion 19 in closely spaced relationship. A biasing means biases the lever part 25 toward the release position. Preferably, the biasing means comprises an extension coil spring 30 mounted at one end to the anvil frame 12 and at the other end to the lever part 25.

A cartridge assembly 32 having proximal 33 and distal 34 ends is mounted in the channel between the lateral side portions 17 and 18 for longitudinal movement relative to the anvil frame 12. The cartridge assembly 32 comprises a cartridge transporting member 35 having first 36 and second 37 side portions that are each elongate in the longitudinal direction and that are spaced to define a ram channel therebetween. The first and second side portions 36 and 37 have surfaces defining a cartridge groove 38 generally adjacent the distal end 34 of the cartridge assembly 32. The cartridge groove surfaces 38 are adapted to releasably receive a cartridge housing 40.

The cartridge housing 40 includes a plurality of staples 41 disposed in rows oriented in planes generally perpendicular to the longitudinal direction and positioned in opposition to the anvil surfaces 23, and manually activatable means, such as a pusher 42, for pressing the staples 41 within longitudinal slots 46 in the cartridge housing 40 against specially shaped anvil surfaces 23 to engage and close the staples 41 in tissue between the cartridge housing 40 and the anvil surfaces 23. The pusher 42 has a pair of edges 43 and is positioned proximate the staples 41 for movement between pre-fired (Figure 12) and fired (Figure 13) positions with the pusher 42 adapted to move distally relative to the cartridge housing 40 when the stapler 10 is fired.

The cartridge housing 40 also has surfaces defining a close fitting hole or alignment through passage 58 positioned in opposition to the alignment aperture 15 in the anvil frame 12. The alignment through passage 58 and the alignment aperture 15 may be generally cylindrical and coaxial and perform a function to be described later.

A means such as pins 44 and grooves 45 mounts the cartridge assembly 32 for longitudinal movement relative to the anvil frame 12 between a closed position (Figures 3, 4 and 5) with the cartridge housing 40 and the anvil surfaces 23 in closely spaced relationship, and an open position (Figures 1 and 2) with the cartridge housing 40 and the anvil surfaces 23 spaced farther from each other than in the closed position.

An elongate T-bar or ram 48 is mounted in the ram channel between the first and second side portions 36 and 37 of the cartridge transport member 35 for longitudinal movement relative to the cartridge transporting member 35 and the anvil frame 12. The T-bar or ram 48 is adapted to engage the pusher 42 to drive the pusher 42 distally to eject the staples 41 from the cartridge housing 40, to press the staples 41 against the specially shaped anvil surfaces 23 and to engage and close the staples 41 in tissues between the cartridge housing 40 and the anvil jaw portion 22 when the cartridge housing 40 and the anvil surfaces 23 are in the closed position.

Figures 2 through 5 sequentially illustrate the operation of the stapler 10. An actuation means 50 operable in a first movement of the lever part 25 from the release to the actuation position initially moves the cartridge assembly 32 from the open to the closed position (Figure 2 and 3). The actuation means 50 is operable in a second movement (Figures 4 and 5) of the lever part 25 from the release to the actuation position to subsequently fire the stapler 10 (e.g. the actuation means 50 drives the ram 48 distally relative to the cartridge transporting member 35 to engage and move the pusher 42 distally to eject the staples 41 from the cartridge housing 40, to press the staples 41 against the anvil surfaces 23 and to engage and close the staples 41 in tissues between the cartridge housing 40 and the anvil jaw portion 22). A means such as a coil spring 51 connected between the anvil frame 12 and the ram 48 bias the cartridge assembly 32 from the closed to the open position and is temporarily overcome by the actuation means 50.

The actuation means 50 preferably comprises a toggle joint linkage 52 having an over center pivoting portion 53 and first 54 and second 55 ends with the first end 54 pivotally mounted to the anvil frame 12 by pin 56 and with the second end 55 pivotally connected to the cartridge transporting member 35 by a pin 57. The over center pivoting portion 53 preferably has surfaces adapted to engage cooperable surfaces on the lever part 25 when the lever part 25 is first moved from the release to the actuation position to move the toggle joint linkage 52 from a retracted position (see Figures 1 and 2 with the cartridge assembly 32 in the open position) past an in-line or centered position with the toggle joint linkage generally straight, to an extended position (Figures 3, 4, 5 and 10) with the toggle joint linkage 52 being slightly bent in a direction inverted relative to the retracted position. Movement of the toggle joint linkage 52 from the retracted to the extended position drives the cartridge assembly 32 from the open to the closed positions.

The actuation means also preferably includes means for retaining the cartridge assembly 32 in the closed position against the bias of the coil spring 51 for biasing the cartridge assembly 32 from the closed to the open position. Preferably, such a means comprises a stop flange 66 on the over center pivoting portion 53 of the toggle joint linkage 52. The stop flange 66 is adapted to engage surfaces on the toggle joint linkage 52 to prevent the toggle joint linkage 52 from moving past the extended position. Alternatively such a means may comprise a stop surface located on the handle housing 24. Also, a relatively weak torsion spring (not shown) may be mounted on the over center pivoting portion 53 of the toggle joint linkage 52 to bias the toggle joint linkage 52 toward the extended position and to prevent the cartridge assembly from accidentally opening when the handle lever 15 is moved from the actuation to the release position just after firing the stapler 10.

The actuation means 50 preferably includes surfaces defining a cam shoulder surface 49 on the ram 48, and a pawl 60 having first 61 and second 62 ends and a cam surface 63 generally adjacent the second end 62. A means such as pin 64 mounts the pawl 60 on the lever part 25 for movement between a first position (Figures 2, 3 and 9) with the cam surface 63 spaced from the cam shoulder surface 49 on the ram 48 and a second position (Figures 4, 10 and 11) with the cam surface 63 engaged with the cam shoulder surface 49 on the ram 48 to afford firing of the stapler 10 by driving the ram 48 distally relative to the cartridge transporting member 35 when the cartridge assembly 32 is in the closed position. Also preferably, the stapler 10 further includes means for biasing the pawl toward the second position such as torsion spring 65, and the ram 48 has sliding surfaces 47 adapted to retain the pawl 60 in the first position until the cartridge assembly 32 is moved from the open to the closed position.

Additionally, the stapler 10 includes a release arm 70 having a first end pivotally mounted to the proximal end 14 of the anvil frame 12 by pin 56 and a second end connected to manually activatable release button 72 extending laterally from the handle housing 24. The release arm 70 has surfaces 73 adapted to engage shoulder portions or "pin" 67 of the pawl 60 and laterally inwardly projecting surfaces (not shown) adapted to engage the over center portion 53 of the toggle joint linkage 52 to initially move the pawl 60 from the second toward the first position and to then move the toggle joint linkage 52 from the extended toward the retracted position to afford movement of the cartridge assembly 32 from the closed to the open position under the bias of spring 51.

The function of the alignment through passage 58 and the alignment aperture 15 will now be described relative to the function of a safety guide member 88 comprising a sleeve 89 having proximal and distal ends. The sleeve 89 is slidably mounted on the anvil frame 12 for longitudinal movement relative thereto. A longitudinally extending alignment or retention pin 90 is mounted on the distal end of the sleeve 89 for movement between an alignment position (Figures 3, 4 and 5) with the alignment pin 90 passing through the cartridge housing alignment through passage 58 and extending into the alignment aperture 15 to position the rows of staples 41 relative to the specially shaped surfaces 23 on the anvil frame 12 to afford a more precisely controlled formation of fired staples and to prevent tissue from escaping from between the cartridge housing 40 and the anvil surfaces 23 when the cartridge assembly 32 is moved to the closed position, and a release position (Figures 1 and 2) with the alignment pin 90 spaced from the alignment through passage 58 and the alignment aperture 15 to afford removal and replacement of the cartridge housing with a new cartridge housing.

In the alignment position, the alignment pin 90 is adapted to position the rows of staples 41 relative to the specially shaped surfaces 23 on the anvil frame 12 to afford a more precisely controlled formation of fired staples and to prevent tissue from escaping from between the cartridge housing 40 and the anvil surfaces 23 when the cartridge assembly 32 is moved to the closed position.

There may also be present means 94 for preventing the cartridge assembly 32 from moving from the open to the closed position unless the alignment pin 90 is in the alignment position, and for preventing the alignment pin 90 from moving from the alignment position toward the release position when the cartridge assembly 32 is in the closed position comprising surfaces defining a safety notch 92 in the cartridge assembly 32 (including both the ram 48 and the cartridge transport member 35) and the anvil frame 12.

The surfaces defining a safety notch 92 in the anvil frame 12, cartridge transport member 35 and ram 48 are aligned when the cartridge assembly 32 is in the open position to define a continuous safety notch (Figures 2, 9, 18 and 19) extending laterally across the stapler 10 and are staggered (see Figures 3, 4 and 10) when the cartridge assembly 32 is in the closed position.

The means 94 includes a safety gate 95 (Figures 18 and 19) having cam shoulder surfaces 85 and return cam surfaces 8 generally opposite the cam surfaces 85. The safety gate 95 includes surfaces defining a safety gate hole 87. The safety gate 95 is mounted adjacent the anvil frame 12 for relative movement thereto between a latched position (Figure 2) with the safety gate 95 engaged with the surfaces defining the continuous safety notch 92 to prevent relative movement between the cartridge assembly 32 and the anvil frame 12 when the cartridge assembly 32 is in the open position, and an unlatched position (Figures 3, 4 and 9) with the safety gate 95 spaced from the safety notch 92 to afford relative longitudinal movement of the cartridge assembly 32 and the anvil frame 12 between the open and closed positions. The handle housing 24 includes guide surfaces defining a slot 77 that affords reciprocating movement of the safety gate 95 between the latched and unlatched positions.

As part of the means 94, the safety guide member 88 includes safety gate deactivating guides 98 projecting proximally from the proximal end of the sleeve 89 and through the safety gate hole 87. Return cam surfaces 8 of the guides 98 bias the safety gate 95 toward the latched position when the alignment pin 90 is in a position other than the alignment position. The cam surfaces 99 at a proximal end of the guides 98 are adapted to engage the cam shoulder surfaces 85 of the safety gate 95 to drive the safety gate 95 from the latched to the unlatched position when the alignment pin 90 is moved to the alignment position. Surfaces 7 (Figure 18) on the cartridge transport member 35 and ram 48 are adapted to engage the "top" of the safety gate 95 to prevent the return cam surfaces 8 from driving the safety gate 95 from the unlatched position toward the latched position when the cartridge assembly is in the closed position. Thus, once the alignment pin 90 is moved to the aligned position, the means 94 also prevents the alignment pin 90 from moving from the alignment position toward the release position when the cartridge assembly 32 is in the closed position.

The stapler 10 according to the present invention also includes a means preventing the cartridge assembly from moving from the open to the closed position unless the stapler 10 is loaded with a ready-to-fire staple cartridge.

As best seen in Figures 12 though 14, a first embodiment of a stapler 10 according to the present invention may comprise a means for preventing the cartridge assembly 32 from moving from the open to the closed position when the stapler 10 is loaded with a cartridge housing 40 having a pusher 42 in a fired position. Such a means prevents approximation and clamping of living tissue between anvil (e.g. 23) and cartridge (e.g. 40) components of the stapler 10 when the stapler 10 is loaded with a spent stapler cartridge, and prevents firing of the stapler 10 when the stapler is loaded with a spent stapler cartridge.

Figures 12 through 14 illustrate a stapler 10 wherein the means for preventing the cartridge assembly 32 from moving from the open to the closed position comprises the anvil frame 12 having surfaces defining a safety aperture 81 opening into the surface of the anvil frame 12 and having a bottom surface, and a locking plate or pin 82 having first 83 and second 84 ends. The locking pin 82 is mounted within the safety aperture 81 for movement between a free travel position (Figure 12) with the first end 83 of the locking pin 82 generally abutting an edge 43 of the pusher 42 to afford a single, reciprocating movement of the cartridge assembly 32 between the open and closed positions, and a blocking position (Figure 13) with the first end 83 of the locking pin 82 projecting beyond the safety aperture 81 and into the path of the ram 48 to prevent movement of the cartridge assembly 32 from the open to the closed position. A biasing means biases the locking pin 82 toward the blocking position. That biasing means preferably comprises a coil spring 86 having a first end connected to the second end 84 of the locking pin 82 and a second end connected to the bottom surf ace of the safety aperture 81.

### OPERATION

The operation of the present invention may now be described with reference to the stapler 10. Figures 2 through 5 sequentially illustrate the operation of the stapler 10.

Figure 2 illustrates the relative positions of the anvil frame 12 and the cartridge assembly 32 in an open position. Typically the stapler 10 may be positioned adjacent the tissue to be stapled, and the alignment pin 90 is then moved from the release position (Figure 2) to the alignment position (Figures 3, 4 and 5) by moving the sleeve 89 distally. When the alignment pin 90 is located in the alignment position, the cam surfaces 99 move the safety gate 95 to the unlatched position to afford relative movement between the cartridge assembly 32 and the anvil frame 12.

In the alignment position, the alignment pin 90 passes through the cartridge housing alignment through passage 58 and extends into the alignment aperture 15 to orient and position the rows of staples 41 relative to the specially shaped surfaces 23 on the anvil frame 12 to afford a more precisely controlled formation of fired staples. Placing the alignment pin 90 in the alignment position also prevents tissue from escaping from between the cartridge housing 40 and the anvil surfaces 23 when the cartridge assembly 32 is moved to the closed position.

Figure 3 illustrates the positions of the anvil frame 12 and the cartridge assembly 32 just after the cartridge assembly 32 is moved to the closed position by a first movement of the lever part 25 from the release position to the actuation position, after which the coil spring 30 returns the lever part 25 to the release position shown in Figure 4. When the cartridge assembly is in the closed position, the surfaces 7 (Figure 18) on the cartridge transport member 35 and ram 48 are adapted to engage the "top" of the safety gate 95 and prevent the return cam surfaces 8 from driving the safety gate 95 from the unlatched position toward the latched position. Thus, once the alignment pin 90 is moved to the aligned position as shown in Figure 3, the means 94 also prevents the alignment pin 90 from moving from the alignment position toward the release position when the cartridge assembly 32 is in the closed position. This feature prevents a user from (1) clamping the stapler 10 on the tissue to be stapled and (2) thereafter moving the alignment pin 90 from the alignment position.

Figure 4 shows the positions of the anvil frame 12 and the cartridge assembly 32 just before the stapler 10 is fired. After the cartridge assembly 32 is moved to the closed position, the pawl 60 moves to the second position with the cam surface 63 generally engaged with the cam shoulder surface 49 of the ram 48. In this position, the stapler 10 is ready to be fired. A second movement of the lever part 25 from the release position to the actuation position (Figure 5) causes the ram 48 to move distally relative to the cartridge transport member 35 and the anvil frame 12, which drives the pusher 42 distally to eject the staples 41 from the cartridge housing 40 to press the staples 41 against the specially shaped anvil surfaces 23 and to engage and close the staples 41 in tissues between the cartridge housing 40 and the anvil jaw portion 22.

Once the cartridge assembly 32 is moved to the closed position, the spring 51 biases the cartridge assembly toward the open position but is prevented from moving the cartridge assembly 32 to the open position by engagement between a stop flange 66 of the toggle joint linkage 52 with another portion of the toggle joint linkage 52 generally adjacent the over center pivoting portion 53. Optionally, engagement between the toggle joint linkage 52 and surfaces on the handle housing 24 may prevent further movement of the toggle joint linkage 52. A torsion spring (not shown) prevents the toggle joint linkage from accidentally "popping-up" or returning to the retracted position when the stapler 10 is fired.

After the stapler 10 is fired, the user may control the return of the cartridge assembly 32 to the open position by moving manually activatable release button 72 "upward" to engage surfaces 73 of release arm 70 with shoulder portions or pin 67 of the pawl 60 and to engage laterally inwardly projecting surfaces (not shown) on the arm 70 with the over center portion 53 of the toggle joint linkage 52 to move the pawl 60 from the second toward the first position and to move the toggle Joint linkage 52 from the extended toward the retracted position against the bias of the torsion spring (not shown). Such a movement of the release arm 70 allows the spring 51 to return the cartridge assembly 32 to the open position.

Once the cartridge assembly 32 is moved to the open position, the return cam surfaces 8 are free to drive the safety gate 95 into engagement with the safety notch 92 to prevent the cartridge assembly 32 from moving from the open to the closed position unless the alignment pin 90 is again moved to the alignment position. Thus this feature operates continuously, even if the stapler is subsequently reused in the same patient.

As best seen in Figure 13, after the stapler 10 is fired and the cartridge assembly 32 is moved to the open position, the pusher 42 is located closer to the cartridge housing 40 than in the pre-fired position so that the second end of pin 82 no longer engages the edge 43 of pusher 42. Thus after the ram 48 moves back to the open position, the spring 86 biases the pin 82 from the free-travel to the blocking position with the first end 83 of the locking pin 82 projecting beyond the safety aperture 81 and into the path of the ram 48 to thereby prevent movement of the cartridge assembly 32 from the open to the closed position. It should be pointed out that the pin 82 will not only prevent the stapler 10 from firing when loaded with a spent cartridge, but will also prevent the firing of the stapler 10 when the stapler is not loaded with a cartridge housing 40 at all.

Also, the pin 82 prevents firing of the stapler 10 should the fired cartridge housing be replaced with another fired cartridge, since the edge portion of the pusher of the fired cartridge would not be able to move the pin 82 from the blocking to the free-travel position. The stapler 10 can be refired only by replacing the fired cartridge housing with an unfired or ready-to-fire cartridge housing 40 having an edge surface 43 of the pusher 42 in the proper position to move the pin 82 to the free-travel position.

It should be noted that the mechanical advantage provided by the toggle joint linkage 52 is at its minimum when the cartridge assembly 32 is in the open position and generally increases as the cartridge assembly 32 moves toward the closed position. Thus, it is important to prevent the cartridge assembly 32 from moving from the open toward the closed position to thereby minimize the shear force transmitted through the toggle joint linkage 52 to the pin 82.

Figures 15 through 17 illustrate a second embodiment of surgical stapler generally designated by the reference character 100 which has many parts that are essentially the same as the parts of the stapler 10 and which have been identified by the same reference numeral to which the suffix "A" has been added. Like the stapler 10, the stapler 100 includes comprises an anvil frame 12A having proximal and distal 16A ends and a pair of lateral side portions 17A and 18A that are each elongate in a longitudinal direction and spaced to define a channel therebetween. The anvil frame 12A has a handle portion (not shown but generally identical to the handle 19 of stapler 10) generally adjacent the proximal end with first and second ends, and a jaw portion 22A having specially shaped anvil surfaces 23A generally adjacent the distal end 16A. The anvil surfaces 23A are positioned in a plane generally perpendicular to the longitudinal direction. The jaw portion 22A includes surfaces defining an alignment aperture 15A opening onto the anvil surfaces 23A.

Like the stapler 10, the stapler 100 includes a cartridge assembly 32A having proximal and distal 34A ends that is mounted in the channel between the lateral side portions 17A and 18A for longitudinal movement relative to the anvil frame 12A. The cartridge assembly 32A comprises a cartridge transporting member 35A having first and second side portions that are each elongate in the longitudinal direction and that are spaced to define a ram channel therebetween. The first and second side portions have surfaces defining a cartridge groove 38A generally adjacent the distal end 34A of the cartridge assembly 32A. The cartridge groove surfaces 38A are adapted to releasably receive a cartridge housing 40A.

The cartridge housing 40A includes a plurality of staples 41A disposed in rows oriented in planes generally perpendicular to the longitudinal direction and positioned in opposition to the anvil surfaces 23A, and manually activatable means, such as a pusher 42A, for pressing the staples 41A within longitudinal slots in the cartridge housing 40A against the specially shaped anvil surfaces 23A to engage and close the staples 41A in tissue between the cartridge housing 40A and the anvil surfaces 23A. The pusher 42A has a pair of edges 43A and is positioned proximate the staples 41A for movement between pre-fired (Figure 15) and fired (Figure 16) positions with the pusher 42A adapted to move distally relative to the cartridge assembly 32A when the stapler 100 is fired. The cartridge housing 40A also has surfaces defining a close fitting hole or alignment through passage positioned in opposition to the alignment aperture 15A in the anvil frame 12A. The alignment through passage and the alignment aperture 15A may be generally cylindrical and coaxial.

An elongate T-bar or ram 48A is mounted in the ram channel between the first and second side portions of the cartridge transport member 35A for longitudinal movement relative to the cartridge transporting member 35A and the anvil frame 12A. The T-bar or ram 48A is adapted to drive the pusher 42A distally to eject the staples 41A from the cartridge housing 40A to press the staples 41A against the specially shaped anvil surfaces 23A and to engage and close the staples 41A in tissues between the cartridge housing 40A and the anvil jaw portion 22A when the cartridge housing 40A the anvil surfaces 23A are in the closed position.

Unlike the stapler 10, the stapler 100 includes a different means 102 for preventing the cartridge assembly 32A from moving from the open to the closed position when the stapler 100 is loaded with a cartridge housing 40A with the pusher 42A in a fired position. Like the means 80, the means 102 prevents approximation and clamping of living tissue between anvil (e.g. 23A) and cartridge (e.g. 40A) components of the stapler 100 when the stapler 100 is loaded with a spent stapler cartridge, and prevents firing of the stapler 100 when the stapler 100 is loaded with a spent stapler cartridge.

The means 102 comprises the anvil frame 12A having surfaces defining a safety aperture 103 opening into the surface of the anvil frame 12A and having a bottom surface, a locking notch 110 in the cartridge transport member 35A of the cartridge assembly 32A, and a locking plate or pin 104 having first 105 and second 106 ends.

The locking pin 104 is mounted within the safety aperture 103 for movement between a free travel position (Figure 15) with the first end 105 of the locking pin 104 generally abutting an edge 43A of the pusher 42A and spaced from the locking notch 110 to afford a single, reciprocating movement of the cartridge assembly 32A between the open and closed positions, and a blocking position (Figure 16) with the first end 105 of the locking pin 104 projecting beyond the safety aperture 103 and engaged with the surfaces of the locking notch 110 to prevent movement of the cartridge assembly 32A from the open to the closed position. Biasing means, such as a coil spring 109 having a first end connected to the second end 106 of the locking pin 104 and a second end connected to the bottom surface of the safety aperture 103, biases the locking pin 104 toward the blocking position.

Figures 20 and 21 illustrate a third embodiment of surgical stapler according to the present invention generally designated by the reference character 200 which has many parts that are essentially the same as the parts of the stapler 100 and which have been identified by the same reference numeral to which the suffix "B" has been added. Like the stapler 100, the stapler 200 includes comprises an anvil frame 12B which is elongate in a longitudinal direction. The anvil frame 12B has a handle portion (not shown but generally identical to the handle 19 of stapler 10), and a jaw portion 22B having specially shaped anvil surfaces (not shown but generally identical to the anvil surfaces 23 and 23A). The anvil surfaces are positioned in a plane generally perpendicular to the longitudinal direction. The jaw portion 22B includes surfaces defining an alignment aperture opening onto the anvil surfaces.

Like the stapler 100, the stapler 200 includes a cartridge assembly 32B that is mounted for longitudinal movement relative to the anvil frame 12B. The cartridge assembly 32B comprises a cartridge transporting member 35B surfaces defining a cartridge groove generally adjacent a distal end of the cartridge assembly 32B. The cartridge groove surfaces are adapted to releasably receive a cartridge housing (not shown but generally identical to the cartridge housings 40 and 40A).

The cartridge housing includes a plurality of staples (not shown but generally identical to the staples 41 and 41) disposed in rows oriented in planes generally perpendicular to the longitudinal direction and positioned in opposition to the anvil surfaces and manually activatable means, such as a pusher 42B, for pressing the staples within longitudinal slots in the cartridge housing against the specially shaped anvil surfaces to engage and close the staples in tissue between the cartridge housing and the anvil surfaces.

The pusher 42A has a pair of edges 43B and is positioned proximate the staples for movement between pre-fired (Figure 20) and fired (Figure 21) positions with the pusher 42B adapted to move distally relative to the cartridge assembly 32B when the stapler 200 is fired.

The cartridge housing also has surfaces defining a close fitting hole or alignment through passage positioned in opposition to an alignment aperture in the anvil frame 12B. The alignment through passage and the alignment aperture may be generally cylindrical and coaxial.

An elongate T-bar or ram 48B is mounted in the ram channel between the first and second side portions of the cartridge transport member for longitudinal movement relative to the cartridge transporting member and the anvil frame 12B. The T-bar or ram 48B is adapted to drive the pusher 42B distally to eject the staples from the cartridge housing to press the staples against the specially shaped anvil surfaces and to engage and close the staples in tissues between the cartridge housing and the anvil jaw portion 22B when the cartridge housing the anvil surfaces are in the closed position.

Unlike the stapler 100, the stapler 200 includes a different means 202 for preventing the cartridge assembly 32B from moving from the open to the closed position unless the stapler 200 is loaded with a ready-to-fire cartridge housing. Like the means 102, the means 202 prevents approximation and clamping of living tissue between anvil and cartridge components of the stapler 200 when the stapler 200 is unloaded or loaded with a spent stapler cartridge, and prevents firing of the stapler 200 when the stapler 200 is unloaded or loaded with a spent stapler cartridge.

Also like the means 102, the means 202 comprises the anvil frame 12B having surfaces defining a safety aperture 103B opening into the surface of the anvil frame 12B and having a bottom surface. Unlike the means 102, the means 202 includes a locking notch 210 in the cartridge transport member of the cartridge assembly 32B having sloped surfaces 220, and a locking plate or pin 204 having first 205 and second 206 ends with a ramped camming surface 230 generally adjacent the first end 205.

The locking pin 204 is mounted within the safety aperture 103B for movement between a free travel position (Figure 20) with the first end 205 of the locking pin 204 generally abutting an edge of the pusher 42B and spaced from the locking notch 210 to afford a single, reciprocating movement of the cartridge assembly 32B between the open and closed positions, and a blocking position (Figure 16) with the first end 205 of the locking pin 204 projecting beyond the safety aperture 103B and engaged with the surfaces of the locking notch 210 to prevent movement of the cartridge assembly 32B from the open to the closed position.

Biasing means such as a coil spring 109B having a first end connected to the second end 206 of the locking pin 204 and a second end connected to the bottom surface of the safety aperture 103B biases the locking pin 204 toward the blocking position.

Optionally, either the locking notch 210 and/or the locking pin 204 may have sloped or ramped camming surfaces to cam the locking pin 204 to the free travel position when the cartridge assembly initially moves from the open to the closed position, but which does not cam the locking pin 204 to the free-travel position when the stapler is unloaded or when the stapler is loaded with a staple cartridge other than a ready-to-fire staple cartridge.

The means 202 is believed to provide a mechanism which is easier to construct than the means 102 as the sloped surfaces 220 and the ramped camming surface 230 are believed to be less sensitive to individual part differences or tolerances, in for example, the relative positions of the cartridge housing 40B, the cartridge transport member 35B and the edge 43B of the pusher 42B.

The present invention has now been described with reference to several embodiments thereof. It will be apparent to those skilled in the art that many changes can be made in the embodiment described without departing from the scope of the present invention. For example, the pin 82 and the spring 86 may be replaced with a plastic part with an integrally molded spring. Also, the pin 104 may be adapted to engage a slot (not shown) in the cartridge housing 40A rather than the locking notch 110 in the cartridge assembly 32. Additionally, the means 94 could be completely eliminated from the stapler 10 according to the present invention, and the anvil frame 12 and the cartridge assembly 32 may be constructed from any suitable material such as but not limited to a metal or plastic material. Moreover, the locking plate or pins 82, 104, 204 may be constructed from any suitable, tough material such as metal or plastic and may have arcuate portions (not shown) adjacent their ends which are adapted to engage the edge (e.g. 43B) of the pusher. The arcuate edges cam the pins toward the free travel position when the stapler is loaded with an unfired cartridge and the cartridge assembly initially moves toward the closed position.

## Claims

1. A surgical stapler comprising:
an elongate anvil including anvil surfaces (23) in a plane generally perpendicular to the direction of elongation of said anvil;
a frame (12) which mounts the anvil;
a cartridge assembly (32) movable relative to said anvil and frame (12) between an open position with the cartridge assembly (32) spaced from said anvil surfaces (23) and a closed position with said cartridge assembly (32) and said anvil surfaces (23) in closely spaced relationship;
said cartridge assembly (32) having a ready to fire disposition and a different fired disposition and including a cartridge housing (40) containing a plurality of staples (41) disposed in at least one row positioned in opposition to said anvil surfaces (23);
means for firing the stapler (10) by engaging and closing the staples (4) in tissue between the cartridge housing (40) and the anvil surfaces (23), and a locking pin or plate (82, 104, 204) for preventing said cartridge assembly (32) from moving from said open to said closed position unless said stapler (10) is loaded with a cartridge in a ready to fire disposition, said locking pin being mounted within the frame (12), movable between a free travel position and a blocking position, biased towards the blocking position, and held against the bias, in the free travel position, by a cartridge in a ready to fire disposition, but not so held by a cartridge in a fired disposition.

2. A stapler as claimed in claim 1, the anvil frame (12) having proximal (14) and distal ends (16) and a pair of lateral side portions (17, 18) each being elongate in a longitudinal direction and spaced to define a channel therebetween, said anvil frame (12) having a handle portion (19) generally adjacent said proximal end (14) and having first (20) and second (21) ends, and a jaw portion (22) having the anvil surfaces generally adjacent said distal end (16).

3. A stapler as claimed in claim 2, in which the means for firing the stapler include an elongate manually movable lever part (25) having first (26) and second (27) ends,
said lever part having pivot means at said second end (27) adapted for affording relative pivotal movement of said lever part (25) and said anvil frame (12) between a release position with said first end (26) of said lever part (25) being spaced from said first end (20) of said handle portion (19) and an actuation position with said lever part (25) and said handle portion (19) in closely spaced relationship; and
biasing means for biasing said lever part toward said release position.

4. A stapler as claimed in claim 2 or 3, characterised by said cartridge assembly (32) having proximal (33) and distal (34) ends and being mounted in said channel between said lateral side portions (17, 18) for longitudinal movement relative to said anvil frame (12);
said cartridge assembly (32) comprising:
a cartridge transporting member (35) having first (36) and second (37) side portions each being elongate in said longitudinal direction and being spaced to define a ram channel therebetween, said first (56) and second (37) side portions having surfaces defining a cartridge groove (38) generally adjacent said distal end (34) of said cartridge assembly (32), said cartridge groove (38) surfaces being adapted to releasably receive a cartridge housing (40) containing a plurality of staples (41) disposed in at least one row positioned in opposition to said anvil surfaces, and pusher means for pressing said staples (41) within said cartridge housing (40) against said anvil surfaces to engage and close the staples (41) in tissue between the cartridge housing (40) and the anvil surfaces, said means for pressing said staples being movable relative to the cartridge housing (40) between a pre-fired and a fired position;
means for mounting said cartridge assembly (40) for longitudinal movement relative to said anvil frame (12) between a closed position with said cartridge housing (40) and said anvil surfaces in closely spaced relationship, and an open position with said cartridge housing (40) and said anvil surfaces spaced farther from each other than in said closed position,
an elongate ram (48) being mounted in said ram channel between said first (36) and said second (37) side portions for longitudinal movement relative to said cartridge transporting member (35) and said anvil frame (12), said ram (48) being adapted for engaging said pusher means to move said pusher means from said prefired to said fired position when said cartridge housing (40) and said anvil surfaces are in said closed position;
actuation means (50) for initially moving said cartridge assembly (32) from said open to said closed position by moving said lever part (25) from said release to said actuation position and for subsequently firing said stapler by moving said ram (48) distally relative to said cartridge transporting member (35) by again moving said lever part (25) from said release to said actuation position, and
means for biasing said cartridge assembly from said closed to said open position.

5. A surgical stapler according to claim 4 wherein said pusher means comprises a pusher (42) having a pair of edges (43) and being positioned proximate the staples (41) for movement between said pre-fired and said fired position with the pusher (42) adapted to move distally relative to said cartridge housing (40) when the stapler is fired, and
said anvil frame (12) having surfaces defining a safety aperture opening (81) into the surface of the anvil frame (12) and having a bottom surface,
said locking pin (82) having first (83) and second (84) ends, said locking pin (82) being mounted within said safety aperture (81) for movement between a free travel position with the first end (83) of the locking pin (82) abutting an edge (43) of said pusher (42) to afford a single, reciprocating movement of said cartridge assembly (32) between said open and closed positions, and a blocking position with the first end (83) of the locking pin (82) projecting beyond the safety aperture (81) to prevent movement of said cartridge assembly (32) from said open to said closed position, and
biasing means for biasing said locking pin (82) toward said blocking position.

6. A surgical stapler according to claim 5 wherein said biasing means for biasing said locking pin (82) toward said blocking position comprises a coil spring (86) having a first end connected to said second end (84) of said locking pin (82) and a second end connected to the bottom surface of said safety aperture (81).

7. A surgical stapler according to claim 4, 5 or 6 wherein said actuation means comprises a toggle joint linkage (52) having an over-centre pivoting portion (53) and first (54) and second ends (55) with the first end (54) fixed to said anvil frame (12) and with the second end (55) connected to said cartridge transporting member (35), said over-centre pivoting portion (53) having surfaces adapted to engage said lever part (25) when said lever part (25) is first moved from said release to said actuation position to move said toggle joint linkage (52) from a retracted position with the cartridge assembly (32) in said open position toward an extended position to drive said cartridge assembly (32) from said open to said closed position,
means for retaining said cartridge assembly (32) in said closed position against the bias of said means for biasing said cartridge assembly (32) from said closed to said open position,
surfaces defining a cam shoulder surface (49) on said ram (48), and
a pawl (10) having first (61) and second (62) ends and a cam surface (163) generally adjacent the second (162) end, and means for mounting said pawl (60) on said lever part (25) for movement between a first position with the cam surface (63) being spaced from the cam shoulder surface (49) on said ram (48) and a second position with the cam surface (63) engaged with the cam shoulder surface (49) on said ram (48) to afford firing of said stapler by driving said ram (48) distally relative to said cartridge transporting member (35) by moving said lever part (25) from said release to said actuation position a second time.

8. A surgical stapler according to claim 7 wherein said stapler further includes means for biasing said pawl (60) toward said second position, and
said ram (48) has surfaces adapted to retain said pawl (60) in said first position until said cartridge assembly (32) is moved from said open to said closed position.

9. A surgical stapler according to claim 7 or 8 wherein said stapler further includes a release arm (70) having a first end pivotally mounted to the proximal end (14) of said anvil frame (12) and a second engagement end, said release arm (70) having surfaces (73) adapted to engage said pawl (60) and said toggle joint linkage (52) to move said pawl (60) from said second to said first position and to move said toggle joint linkage (52) from said extended toward said retracted position to afford movement of said cartridge assembly (32) from said closed to said open position under the bias of said means for biasing said cartridge assembly (32) from said closed to said open position.

10. A surgical stapler according to any one of claims 5 to 9 wherein said locking pin (82) has a ramped camming surface (230) generally adjacent to its first end.

11. A stapler as claimed in any one of the preceding claims wherein the preventing means senses whether said stapler is loaded with a fired cartridge housing, for preventing the stapler from being fired when loaded with said fired cartridge housing.

12. A stapler according to any one of claims 5 to 10 wherein said cartridge assembly (32) includes surfaces defining a locking notch (110, 210), and, in the blocking position, said locking pin (82) projects beyond the safety aperture (81) and engages with the surfaces of the locking notch (110, 210) to prevent movement of the cartridge assembly (32) from the open to the closed position.

13. A stapler according to claim 12 wherein said locking notch (110, 210) includes a ramped surface (220) for camming the locking pin.

14. A stapler as claimed in any one of the preceding claims, and including a safety guide member (88) comprising:
a sleeve (89) having proximal and distal ends and being slidably mounted on said anvil frame (12) for longitudinal movement relative thereto,
a longitudinally extending alignment or retention pin (90) mounted on said distal end of said sleeve (89) for movement between an alignment position with said pin (90) passing through said cartridge housing alignment through passage (58) and extending into said alignment aperture (15) to position said rows of staples (41) relative to said specially shaped surfaces (23) on said anvil frame (12) to afford a more precisely controlled formation of fired staples and to prevent tissue from escaping from between the cartridge housing (40) and the anvil surfaces (23) when the cartridge assembly (32) is moved to the closed position, and a release position with said alignment pin (90) spaced from said alignment through passage (58) and said alignment aperture (15) to afford removal and replacement of said cartridge housing (40) with a new cartridge housing, and means (94), mounted on said safety guide member (88), for repeatedly and continuously preventing said cartridge assembly (32) from moving from said open to said closed position unless said alignment pin (90) is in said alignment position.

15. A stapler as claimed in claim 14 wherein the means (94) for repeatedly and continuously preventing said cartridge assembly (32) from moving from said open to said closed position unless said alignment pin (90) is in said alignment position comprises:
said anvil frame (12), cartridge transport member (35) and ram (48) each having surfaces defining safety notches (92) which are aligned when said cartridge assembly (32) is in said open position,
a safety gate (95) having cam shoulder surfaces (85) and surfaces defining a hole, said safety gate (95) being mounted adjacent said anvil frame (12) for relative movement thereto between a latched position with the safety gate (95) engaged with the surfaces defining the safety notches (92) to prevent relative movement between the cartridge assembly (32) and the anvil frame (12) when the cartridge assembly (32) is in said open position, and an unlatched position with the safety gate (95) spaced from said safety notches (92) to afford relative movement of the cartridge assembly (32) and the anvil frame (12) between the open and closed positions, and
said safety guide member (88) including safety gate deactivating guides (98) projecting proximally from the proximal end of said sleeve (89) and through said safety gate hole (87), said guides (98) being adapted to drive said safety gate (95) toward said latched position when the alignment pin (90) is in a position other than the alignment position and having cam surfaces (99) at a proximal end adapted to engage the cam shoulder surfaces (85) of said safety gate (95) to drive the safety gate (95) from said latched to said unlatched position when the alignment pin (90) is moved to the alignment position.

16. A stapler according to claim 15 wherein said stapler includes means (94) for preventing said alignment pin (90) from moving from said alignment position toward said release position when said cartridge assembly (32) is in said closed position comprising:
said safety gate deactivating guides (98) having return cam surfaces (8) generally opposite said cam surfaces (85) adapted to engage surfaces on said safety gate (95) to drive the safety gate (95) from the unlatched toward the latched position when the alignment pin (90) is moved away from the alignment position, and
surfaces (7) on said cartridge transport member (35) and ram (48) adapted to engage the safety gate (95) to prevent the alignment pin (90) from moving from the alignment position toward the release position when the cartridge assembly (32) is in the closed position.

## Patentansprüche

1. Chirurgische Klammereinrichtung umfassend:
einen langgestreckten Anschlag, der Anschlagoberflächen (23) in einer Ebene aufweist, die im allgemeinen senkrecht zur Längsrichtung des Anschlages ist;
einen Rahmen (12), der den Anschlag befestigt;
einen Magazinaufbau (32), der relativ zum Anschlag und Rahmen (12) zwischen einer offenen Position mit dem Magazinaufbau (32) von den Anschlagoberflächen (23) beabstandet und einer geschlossenen Position mit dem Magazinaufbau (32) und den Anschlagoberflächen (23) in eng beabstandeter Beziehung bewegbar ist;
wobei der Magazinaufbau (32) eine abschußbereite Anordnung und eine unterschiedliche, abgeschossene Anordnung besitzt und ein Magazingehäuse (40) aufweist, das eine Mehrzahl von Klammern (41) enthält, die in zumindest einer Reihe angeordnet sind, die entgegengesetzt den Anschlagoberflächen (23) angeordnet sind;
eine Einrichtung, um die Klammereinrichtung (10) durch das Ineingrifftreten und Schließen der Klammern (4) in Gewebe zwischen dem Magazingehäuse (40) und den Anschlagoberflächen (23) abzuschießen, und einen Sperrstift oder eine Platte (82, 104, 204), um den Magazinaufbau (32) daran zu hindern, sich von der offenen in die geschlossene Position zu bewegen, wenn nicht die Klammereinrichtung (10) mit einem Magazin in einer abschußbereiten Anordnung bestückt ist, wobei der Verriegelungsstift innerhalb des Rahmens (12) montiert ist und zwischen einer frei beweglichen Position und einer Sperrposition bewegbar ist, in Richtung der Sperrposition vorgespannt ist und gegen die Vorspannung in der frei beweglichen Position durch ein abschußbereites Magazin, aber nicht durch ein Magazin in einer abgeschossenen Anordnung gehalten wird.

2. Klammereinrichtung gemäß Anspruch 1, wobei der Anschlagrahmen (12) proximale (14) und distale (16) Enden und ein Paar von seitlichen Seitenbereichen (17, 18) besitzt, die beide in einer Längsrichtung langgestreckt und beabstandet sind, um eine Rinne dazwischen zu begrenzen, wobei der Anschlagrahmen (12) einen Griffbereich (19) im allgemeinen neben dem proximalen Ende (14) besitzt und erste (20) und zweite (21) Enden besitzt, und ein Klemmbackenbereich (22) mit den Anschlagoberflächen im allgemeinen neben dem distalen Ende (16).

3. Klammereinrichtung gemäß Anspruch 2, bei der die Einrichtung zum Abschießen der Klammereinrichtung ein langgestrecktes, von Hand bewegbares Hebelteil (25) mit ersten (26) und zweiten (27) Enden,
wobei das Hebelteil eine Schwenkeinrichtung am zweiten Ende (27) besitzt, die dazu geeignet ist, um eine relative Schwenkbewegung des Hebelteils (25) und des Anschlagrahmens (12) zwischen einer Löseposition mit dem ersten Ende (26) des Hebelteils (25) von dem ersten Ende (20) des Griffbereichs (19) beabstandet und einer Betätigungsposition mit dem Hebelteil (25) und dem Griffbereich (19) in eng beabstandeter Beziehung zu ermöglichen; und
eine Vorspanneinrichtung, um das Hebelteil in Richtung der Löseposition vorzuspannen, aufweist.

4. Klammereinrichtung gemäß Anspruch 2 oder 3, dadurch gekennzeichnet, daß der Magazinaufbau (32) proximale (33) und distale (34) Enden besitzt und in der Rinne zwischen den seitlichen Seitenbereichen (17, 18) für eine Längsbewegung relativ zum Anschlagrahmen (12) montiert ist,
wobei der Magazinaufbau (32) umfaßt:
ein Magazintransportelement (35) mit ersten (36) und zweiten (37) Seitenbereichen, die beide in der Längsrichtung langgestreckt sind und beabstandet sind, um eine Stößelrinne dazwischen zu begrenzen, wobei die ersten (36) und zweiten (37) Seitenbereiche Oberflächen besitzen, die eine Magazinnut (38) im allgemeinen neben dem distalen Ende (34) des Magazinaufbaus (32) begrenzen, wobei die Oberflächen der Magazinnut (38) dazu geeignet sind, lösbar ein Magazingehäuse (40) aufzunehmen, das eine Mehrzahl von Klammern (41) enthält, die in zumindest einer Reihe entgegengesetzt den Anschlagoberflächen positioniert angeordnet sind, und eine Schiebereinrichtung, um die Klammern (41) innerhalb des Magazingehäuses (40) gegen die Anschlagoberflächen zu drücken, um die Klammern (41) im Gewebe zwischen dem Magazingehäuse (40) und den Anschlagoberflächen in Eingriff zu bringen und zu schließen, wobei die Einrichtung, um die Klammern zu drücken, relativ zum Magazingehäuse (40) zwischen einer Position vor dem Abschießen und einer abgeschossenen Position bewegbar ist;
eine Einrichtung, um den Magazinaufbau (40) für eine Längsbewegung relativ zu dem Anschlagrahmen (12) zwischen einer geschlossenen Position mit dem Magazingehäuse (40) und den Anschlagoberflächen in eng beabstandeter Beziehung und einer offenen Position mit dem Magazingehäuse (40) und den Anschlagoberflächen weiter voneinander beabstandet als in der geschlossenen Position anzubringen,
einen langgestreckten Stößel (48), der in der Stößelrinne zwischen den ersten (36) und den zweiten (37) Seitenbereichen für eine Längsbewegung relativ zum Magazintransportelement (35) und dem Anschlagrahmen (12) angebracht ist, wobei der Stößel (48) dazu geeignet ist, mit der Schiebereinrichtung in Eingriff zu treten und die Schiebereinrichtung von der Position vor dem Abschießen in die abgeschossene Position zu bewegen, wenn das Magazingehäuse (40) und die Anschlagoberflächen in der geschlossenen Position sind;
eine Betätigungseinrichtung (50), um anfänglich den Magazinaufbau (32) von der offenen in die geschlossene Position durch das Bewegen des Hebelteils (25) von der Löse- in die Betätigungsposition zu bewegen und für das nachfolgende Abschießen der Klammereinrichtung durch das Bewegen des Stößels (48) in distaler Richtung relativ zu dem Magazintransportelement (35), indem wieder das Hebelteil (25) von der Löse- in die Betätigungsposition bewegt wird, und
eine Einrichtung, um den Magazinaufbau von der geschlossenen in die offene Position vorzuspannen.

5. Chirurgische Klammereinrichtung gemäß Anspruch 4, wobei die Schiebereinrichtung einen Schieber (42) umfaßt mit einem Paar von Kanten (43) und neben den Klammern (41) positioniert für eine Bewegung zwischen der Position vor dem Abschießen und der abgeschossenen Position, wobei der Schieber (42) dazu geeignet ist, sich in distaler Richtung relativ zum Magazingehäuse (40) zu bewegen, wenn die Klammereinrichtung abgeschossen wird, und
wobei der Anschlagrahmen (12) Oberflächen besitzt, die eine Sicherheitsöffnung (81) begrenzen, die sich in die Oberfläche des Anschlagrahmens (12) öffnet und eine untere Oberfläche besitzt;
wobei der Sperrstift (82) erste (83) und zweite (84) Enden besitzt und innerhalb der Sicherheitsöffnung (81) für eine Bewegung zwischen einer frei beweglichen Position mit dem ersten Ende (83) des Sperrstiftes (82) gegen eine Kante (43) des Schiebers (42) anstoßend, um eine einzelne, hin- und hergerichtete Bewegung des Magazinaufbaus (32) zwischen der offenen und geschlossenen Position zu ermöglichen, und einer Sperrposition angebracht ist, bei der das erste Ende (83) des Sperrstiftes (82) über die Sicherheitsöffnung (81) hinaus vorsteht, um eine Bewegung des Magazinaufbaus (32) von der offenen in die geschlossene Position zu verhindern, und
eine Vorspanneinrichtung, um den Sperrstift (82) in Richtung der Sperrposition vorzuspannen.

6. Chirurgische Klammereinrichtung gemäß Anspruch 5, wobei die Vorspanneinrichtung, um den Sperrstift (82) in Richtung der Sperrposition vorzuspannen, eine Schraubenfeder (86) umfaßt mit einem ersten Ende, das mit dem zweiten Ende (84) des Sperrstiftes (82) verbunden ist, und einem zweiten Ende, das mit der unteren Oberfläche der Sicherheitsöffnung (81) verbunden ist.

7. Chirurgische Klammereinrichtung gemäß Anspruch 4, 5 oder 6, wobei die Betätigungseinrichtung eine Kniegelenkverbindung (52) umfaßt, mit einem Übertotpunkt-Schwenkbereich (53) und ersten (54) und zweiten (55) Enden, wobei das erste Ende (54) am Anschlagrahmen (12) befestigt ist und das zweite Ende (55) mit dem Magazintransportelement (35) verbunden ist, wobei der Übertotpunkt-Schwenkbereich (53) Oberflächen besitzt, die dazu geeignet sind, mit dem Hebelteil (25) in Eingriff zu treten, wenn das Hebelteil (25) zuerst von der Löse- in die Betätigungsposition bewegt wird, um die Kniegelenkverbindung (52) von einer zurückgezogenen Position mit dem Magazinaufbau (32) in der offenen Position in Richtung einer ausgefahrenen Position zu bewegen, um den Magazinaufbau (32) von der offenen in die geschlossene Position zu bewegen,
eine Einrichtung, um den Magazinaufbau (32) in der geschlossenen Position gegen die Vorspannung der Einrichtung zum Vorspannen des Magazinaufbaus (32) von der geschlossenen in die offene Position zu halten,
Oberflächen, die eine Nockenschulteroberfläche (49) auf dem Stößel (48) definieren, und
eine Klinke (12) mit ersten (61) und zweiten (62) Enden und einer Nockenoberfläche (163) im allgemeinen neben dem zweiten (162) Ende, und eine Einrichtung, um die Klinke (60) auf dem Hebelteil (25) für eine Bewegung zwischen einer ersten Position mit der Nockenoberfläche (63) von der Nockenschulteroberfläche (49) auf dem Stößel (48) beabstandet und einer zweiten Position mit der Nockenoberfläche (63) in Eingriff mit der Nockenschulteroberfläche (49) auf dem Stößel (48) anzubringen, um das Abschießen der Klammereinrichtung durch das Bewegen des Stößels (48) in distaler Richtung relativ zum Magazintransportelement (35) durch das Bewegen ein zweites Mal des Hebelteils (25) von der Löse- in die Betätigungsposition zu ermöglichen.

8. Chirurgische Klammereinrichtung gemäß Anspruch 7, wobei die Klammereinrichtung weiterhin eine Einrichtung aufweist, um die Klinke (60) in Richtung der zweiten Position vorzuspannen und
der Stößel (48) Oberflächen besitzt, die dazu geeignet sind, die Klinke (60) in der ersten Position zu halten, bis der Magazinaufbau (32) von der offenen in die geschlossene Position bewegt wird.

9. Chirurgische Klammereinrichtung gemäß Anspruch 7 oder 8, wobei die Klammereinrichtung weiterhin einen Lösearm (70) aufweist mit einem ersten Ende, das schwenkbar am proximalen Ende (14) des Anschlagrahmens (12) angebracht ist, und einem zweiten Eingriffsende, wobei der Lösearm (70) Oberflächen (73) besitzt, die dazu geeignet sind, mit der Klinke (60) und der Kniegelenkverbindung (52) in Eingriff zu treten, um die Klinke (60) von der zweiten in die erste Position zu bewegen und die Kniegelenkverbindung (52) von der ausgefahrenen in Richtung der zurückgezogenen Position zu bewegen, um eine Bewegung des Magazinaufbaus (32) von der geschlossenen in die offene Position unter der Vorspannung der Einrichtung zum Vorspannen des Magazinaufbaus (32) von der geschlossenen in die offene Position zu ermöglichen.

10. Chirurgische Klammereinrichtung gemäß einem der Ansprüche 5 bis 9, wobei der Sperrstift (82) eine rampenförmige Nockenoberfläche (230) im allgemeinen neben seinem ersten Ende besitzt.

11. Klammereinrichtung gemäß einem der vorhergehenden Ansprüche, wobei die Einrichtung zum Verhindern erfaßt, ob die Klammereinrichtung mit einem abgeschossenen Magazingehäuse bestückt ist, um die Klammereinrichtung daran zu hindern, abgeschossen zu werden, wenn sie mit dem abgeschossenen Magazingehäuse bestückt ist.

12. Klammereinrichtung gemäß einem der Ansprüche 5 bis 10, wobei der Magazinaufbau (32) Oberflächen aufweist, die eine Sperrnut (110, 210) begrenzen und, wobei, in der Sperrposition, der Sperrstift (82) über die Sicherheitsöffnung (81) hinaus vorsteht und mit den Oberflächen der Sperrnut (110, 210) in Eingriff tritt, um eine Bewegung des Magazinaufbaus (32) von der offenen in die geschlossene Position zu verhindern.

13. Klammereinrichtung gemäß Anspruch 12, wobei die Sperrnut (110, 210) eine rampenförmige Oberfläche (220) zum nockenartigen Verschieben des Sperrstiftes aufweist.

14. Klammereinrichtung gemäß einem der vorhergehenden Ansprüche und umfassend ein Sicherheitsführungselement (88) umfassend:
eine Hülse (89) mit proximalen und distalen Enden, die verschiebbar auf dem Anschlagrahmen (12) für eine Längsbewegung relativ zu diesem montiert ist,
einen sich in Längsrichtung erstreckenden Ausricht- oder Haltestift (90), der auf dem distalen Ende der Hülse (89) für eine Bewegung montiert ist zwischen einer Ausrichtposition mit dem Stift (90), der durch die Magazingehäuseausrichtung durch den Durchgang (58) hindurchtritt und sich in die Ausrichtöffnung (15) erstreckt, um die Reihen von Klammern (41) relativ zu den speziell geformten Oberflächen (23) auf dem Anschlagrahmen (12) auszurichten, um eine präziser geregelte Verformung der abgeschossenen Klammern zu ermöglichen und Gewebe daran zu hindern, zwischen dem Magazingehäuse (40) und den Anschlagoberflächen auszutreten, wenn der Magazinaufbau (32) in die geschlossene Position bewegt ist, und eine Löseposition mit dem Ausrichtstift (90) von der Ausrichtung durch den Durchgang (58) und der Ausrichtöffnung (15) beabstandet, um ein Entfernen und Ersetzen des Magazingehäuses (40) durch ein neues Magazingehäuse zu ermöglichen, und eine Einrichtung (94), die auf dem Sicherheitsführungselement (88) angebracht ist, um wiederholt und dauerhaft den Magazinaufbau (32) daran zu hindern, sich von der offenen in die geschlossene Position zu bewegen, wenn der Ausrichtstift (90) nicht in der Ausrichtposition ist.

15. Klammereinrichtung gemäß Anspruch 14, wobei die Einrichtung (94) zum wiederholten und dauerhaften Verhindern, daß sich der Magazinaufbau (32) von der offenen in die geschlossene Position bewegt, wenn der Ausrichtstift (90) nicht in der Ausrichtposition ist, umfaßt:
den Anschlagrahmen (12), das Magazintransportelement (35) und den Stößel (48), die alle Oberflächen besitzen, die Sicherheitsnuten (92) begrenzen, die ausgerichtet sind, wenn der Magazinaufbau (32) in der offenen Position ist,
eine Sicherheitsklappe (95) mit Nockenschulteroberflächen (85) und Oberflächen, die eine Öffnung begrenzen, wobei die Sicherheitsklappe (95) neben dem Anschlagrahmen (12) montiert ist für eine relative Bewegung zu diesem zwischen einer verriegelten Position mit der Sicherheitsklappe (95) in Eingriff mit den Oberflächen, welche die Sicherheitsnuten (92) begrenzen, um eine relative Bewegung zwischen dem Magazinaufbau (32) und dem Anschlagrahmen (12) zu verhindern, wenn der Magazinaufbau (32) in der offenen Position ist, und einer gelösten Position, wobei die Sicherheitsklappe (95) von den Sicherheitsnuten (92) beabstandet ist, um eine relative Bewegung des Magazinaufbaus (32) und des Anschlagrahmens (12) zwischen der offenen und geschlossenen Position zu ermöglichen, und
wobei das Sicherheitsführungselement (88) Deaktivierführungen (98) für die Sicherheitsklappe aufweist, die in proximaler Richtung vom proximalen Ende der Hülse (89) und durch die Sicherheitsklappenöffnung (87) vorstehen, wobei die Führungen (98) dazu geeignet sind, die Sicherheitsklappe (95) in Richtung der verriegelten Position zu bewegen, wenn der Ausrichtstift (90) in einer anderen als der Ausrichtposition ist, und Nockenoberflächen (99) an einem proximalen Ende besitzt, die dazu geeignet sind, mit den Nockenschulteroberflächen (85) der Sicherheitsklappe (95) in Eingriff zu treten, um die Sicherheitsklappe (95) von der verriegelten in die gelöste Position zu bewegen, wenn der Ausrichtstift (90) in die Ausrichtposition bewegt wird.

16. Klammereinrichtung gemäß Anspruch 15, wobei die Klammereinrichtung Einrichtungen (94) aufweist, um den Ausrichtstift (90) daran zu hindern, sich von der Ausrichtposition in die Richtung der Löseposition zu bewegen, wenn der Magazinaufbau (32) in der geschlossenen Position ist, umfassend:
Deaktivierführungen (98) für die Sicherheitsklappe mit Rückkehrnockenoberflächen (8), die im allgemeinen den Nockenoberflächen (95) gegenüberliegen und dazu geeignet sind, mit Oberflächen auf der Sicherheitsklappe (95) in Eingriff zu treten, um die Sicherheitsklappe (95) von der entriegelten in Richtung der gesperrten Position zu bewegen, wenn der Ausrichtstift (90) von der Ausrichtposition weg bewegt wird, und
Oberflächen (7) auf dem Magazintransportelement (35) und Stößel (48), die dazu geeignet sind, mit der Sicherheitsklappe (95) in Eingriff zu treten, um den Ausrichtstift (90) daran zu hindern, sich von der Ausrichtposition in Richtung der Löseposition zu bewegen, wenn der Magazinaufbau (32) in der geschlossenen Position ist.

## Revendications

1. Agrafeuse chirurgicale comprenant :
une enclume oblongue incluant des surfaces d'enclume (23) dans un plan généralement perpendiculaire à la direction d'allongement de ladite enclume ;
un châssis (12) sur lequel est monté l'enclume ;
un ensemble de cartouche (32) déplaçable relativement auxdits enclume et châssis (12) entre une Position ouverte, où l'ensemble de cartouche (32) est espacé desdites surfaces d'enclume (23) et une position fermée où ledit ensemble de cartouche (32) et lesdites surfaces d'enclume (23) sont dans une relation faiblement espacée ;
ledit ensemble de cartouche (32) ayant une disposition prête à tirer et une disposition tirée différente et incluant un boitier de cartouche (40) contenant une pluralité d'agrafes (41) disposées selon au moins une rangée, positionnées pour être opposées auxdites surfaces d'enclume (23) ;
un moyen pour tirer avec l'agrafeuse (10) en engageant et en fermant les agrafes (4) dans le tissu entre le boitier de cartouche (40) et les surfaces d'enclume (23), et un axe ou plaque de verrouillage (82, 104, 204) pour empêcher que ledit ensemble de cartouche (32) se déplace de ladite position ouverte à ladite position fermée à moins que ladite agrafeuse (10) ne soit chargée avec une cartouche dans une disposition prête à tirer, ledit axe de verrouillage étant installé dans le châssis (12) déplaçable entre une position de déplacement libre et une position de blocage, sollicité vers la position de blocage et retenu contre la sollicitation, dans la position de déplacement libre, par une cartouche dans une disposition prête à tirer, mais n'étant pas ainsi retenu par une cartouche dans une disposition tirée.

2. Agrafeuse selon la revendication 1, où le châssis d'enclume (12) a des extrémités proximale (14) et distale (16) et une paire de portions latérales (17, 18), chacune allongée dans une direction longitudinale et espacées pour définir un canal entre celles-ci, ledit châssis d'enclume (12) ayant une portion de poignée (19) généralement adjacente à ladite extrémité proximale (14) et ayant des première (20) et seconde (21) extrémités, et une portion de mâchoire (22) ayant les surfaces d'enclume généralement adjacentes à ladite extrémité distale (16).

3. Agrafeuse selon la revendication 2, où le moyen pour tirer avec l'agrafeuse comprend une partie de levier oblongue (25) manuellement déplaçable avec des première (26) et seconde (27) extrémités,
ladite partie de levier ayant un moyen pivotant à ladite seconde extrémité (27) apte à permettre un mouvement pivotant relatif de ladite partie de levier (25) et dudit châssis d'enclume (12) entre une position de relâchement où ladite première extrémité (26) de ladite partie de levier (25) est espacée de ladite première extrémité (20) de ladite portion de poignée (19) et une position d'actionnement où ladite partie de levier (25) et ladite partie de poignée (19) sont dans une relation faiblement espacée ; et
un moyen de sollicitation pour solliciter ladite partie de levier vers ladite position de relâchement.

4. Agrafeuse selon la revendication 2 ou 3, caractérisée en ce que ledit ensemble de cartouche (32) a des extrémités proximale (33) et distale (34) et est installé dans ledit canal entre lesdites portions latérales (17, 18) en vue d'un mouvement longitudinal relativement audit châssis d'enclume (12) ;
ledit ensemble de cartouche (32) comprenant :
un élément de transport de cartouche (35) ayant des première (36) et seconde (37) portions latérales dont chacune est allongée dans ladite direction longitudinale et qui sont espacées pour définir un canal de plongeur entre celles-ci, lesdites première (56) et seconde (37) portions latérales ayant des surfaces définissant une rainure de cartouche (38) généralement adjacente à ladite extrémité distale (34) dudit ensemble de cartouche (32), lesdites surfaces de rainure de cartouche (38) étant aptes à recevoir relâchablement un boîtier de cartouche (40) contenant une pluralité d'agrafes (41) disposées selon au moins une rangée, positionnées pour être opposées auxdites surfaces d'enclume, et un moyen formant poussoir pour pousser lesdites agrafes (41) dans ledit boîtier de cartouche (40) contre lesdites surfaces d'enclume pour engager et fermer les agrafes (41) dans le tissu entre le boîtier de cartouche (40) et les surfaces d'enclume, ledit moyen pour pousser lesdites agrafes étant déplaçable relativement au boîtier de cartouche (40) entre une position avant le tir et une position après le tir ;
un moyen pour installer ledit ensemble de cartouche (40) en vue d'un mouvement longitudinal relativement audit châssis d'enclume (12) entre une position fermée où ledit boîtier de cartouche (40) et lesdites surfaces d'enclume sont dans une relation faiblement espacée, et une position ouverte où ledit boitier de cartouche (40) et lesdites surfaces d'enclume sont espacées davantage l'une de l'autre que dans ladite position fermée,
un plongeur oblong (48) étant installé dans ledit canal de plongeur entre lesdites première (36) et seconde (37) portions latérales en vue d'un mouvement longitudinal relativement audit élément de transport de cartouche (35) et audit châssis d'enclume (12), ledit plongeur (48) étant apte à venir en prise avec ledit moyen formant poussoir pour déplacer ledit moyen formant poussoir de ladite position avant le tir à ladite position tirée lorsque ledit boitier de cartouche (40) et lesdites surfaces d'enclume se trouvent dans ladite position fermée ;
un moyen d'actionnement (50) pour déplacer initialement ledit ensemble de cartouche (32) de ladite position ouverte à ladite position fermée en déplaçant ladite partie de levier (25) de ladite position de relâchement à ladite position d'actionnement et pour tirer ensuite avec ladite agrafeuse en déplaçant ledit plongeur (48) distalement relativement audit élément de transport de cartouche (35) en déplaçant de nouveau ladite partie de levier (25) de ladite position de relâchement à ladite position d'actionnement, et
un moyen pour solliciter ledit ensemble de cartouche de ladite position fermée à ladite position ouverte.

5. Agrafeuse chirurgicale selon la revendication 4, où ledit moyen formant poussoir comprend un poussoir (42) présentant une paire de bords (43) et étant positionné à proximité des agrafes (41) pour un déplacement entre ladite position avant le tir et ladite position tirée, où le poussoir (42) est apte à se déplacer distalement relativement audit boîtier de cartouche (40) lorsqu'on tire avec l'agrafeuse et
ledit châssis d'enclume (12) ayant des surfaces définissant une ouverture de sécurité (81) s'ouvrant dans la surface du châssis d'enclume (12) et ayant une surface de fond,
ledit axe de verrouillage (82) ayant des première (83) et seconde (84) extrémités, ledit axe de verrouillage (82) étant installé dans ladite ouverture de sécurité (81) en vue d'un déplacement entre une position de déplacement libre où la première extrémité (83) de l'axe de verrouillage (82) bute contre un bord (43) dudit poussoir (42) pour permettre un seul mouvement de va-et-vient dudit ensemble de cartouche (32) entre lesdites positions ouverte et fermée, et une position de blocage où la première extrémité (83) de l'axe de verrouillage (82) fait saillie au-delà de l'ouverture de sécurité (81) pour empêcher un déplacement dudit ensemble de cartouche (32) de ladite Position ouverte à la position fermée, et un moyen de sollicitation pour solliciter ledit axe de verrouillage (82) vers ladite position de blocage.

6. Agrafeuse chirurgicale selon la revendication 5, où ledit moyen de sollicitation pour solliciter ledit axe de verrouillage (82) vers ladite position de blocage comprend un ressort hélicoïdal (86) dont une première extrémité est reliée à ladite seconde extrémité (84) dudit axe de verrouillage (82) et dont une seconde extrémité est reliée à la surface de fond de ladite ouverture de sécurité (81).

7. Agrafeuse chirurgicale selon la revendication 4, 5 ou 6, où ledit moyen d'actionnement comprend un levier à genouillère (52) ayant une portion pivotante à passage brusque (53) et des première (54) et seconde (55) extrémités, où la première extrémité (54) est fixée audit châssis d'enclume (12) et où la seconde extrémité (55) est reliée audit élément de transport de cartouche (35), ladite portion pivotante à passage brusque (53) ayant des surfaces aptes à venir en prise avec ladite partie de levier (25) lorsque ladite partie de levier (25) est d'abord déplacée de ladite position de relâchement à ladite position d'actionnement pour amener ledit levier à genouillère (52) d'une position rétractée où l'ensemble de cartouche (32) se trouve dans ladite position ouverte vers une position étendue pour entraîner ledit ensemble de cartouche (32) de ladite position ouverte à ladite position fermée,
un moyen pour retenir ledit ensemble de cartouche (32) dans ladite position fermée contre la sollicitation dudit moyen pour solliciter ledit ensemble de cartouche (32) de ladite position fermée à ladite position ouverte,
des surfaces définissant une surface d'épaulement de came (49) sur ledit plongeur (48), et
un cliquet (10) ayant des première (61) et seconde (62) extrémités et une surface de came (163) généralement adjacente à la seconde (162) extrémité et un moyen pour monter ledit cliquet (60) sur ladite partie de levier (25) en vue d'un déplacement entre une première position où la surface de came (63) est espacée de la surface d'épaulement de came (49) sur ledit plongeur (48) et une seconde position où la surface de came (63) est en prise avec la surface d'épaulement de came (49) sur ledit plongeur (48) pour permettre le tir avec ladite agrafeuse en entraînant ledit plongeur (48) distalement relativement audit élément de transport de cartouche (35) en déplacant ladite partie de levier (25) de ladite position de relâchement à ladite position d'actionnement une deuxième fois.

8. Agrafeuse chirurgicale selon la revendication 7, où ladite agrafeuse comprend en outre un moyen pour solliciter ledit cliquet (60) vers ladite seconde position et
ledit plongeur (48) a des surfaces conçues pour retenir ledit cliquet (60) dans ladite première position jusqu'à ce que ledit ensemble de cartouche (32) soit déplacé de ladite position ouverte à ladite position fermée.

9. Agrafeuse chirurgicale selon la revendication 7 ou 8, où ladite agrafeuse comporte en outre un bras de relâchement (70) ayant une première extrémité montée d'une manière pivotante sur l'extrémité proximale (14) dudit châssis d'enclume (12) et une seconde extrémité d'engagement, ledit bras de relâchement (70) ayant des surfaces (73) aptes à venir en prise avec ledit cliquet (60) et ledit levier à genouillère (52) pour déplacer ledit cliquet (60) de ladite seconde position à ladite première position et pour déplacer ledit levier à genouillère (52) de ladite position étendue vers ladite position rétractée pour permettre le déplacement dudit ensemble de cartouche (32) de ladite position fermée à ladite position ouverte sous la sollicitation dudit moyen pour solliciter ledit ensemble de cartouche (32) de ladite position fermée à ladite position ouverte.

10. Agrafeuse chirurgicale selon l'une des revendications 5 à 9, où ledit axe de verrouillage (82) a une surface de came formant rampe (230) généralement adjacente à sa première extrémité.

11. Agrafeuse selon l'une des revendications précédentes, où le moyen d'empêchement détecte si ladite agrafeuse est chargée avec un boîtier de cartouche tiré, pour empêcher un tir avec l'agrafeuse lorsque celle-ci est chargée avec ledit boîtier de cartouche tiré.

12. Agrafeuse selon l'une des revendications 5 à 10, où ledit ensemble de cartouche (32) inclut des surfaces définissant une encoche de verrouillage (110, 210) et, dans la position de blocage, ledit axe de verrouillage (82) fait saillie au-delà de l'ouverture de sécurité (81) et vient en prise avec les surfaces de l'encoche de verrouillage (110, 210) pour empêcher un déplacement de l'ensemble de cartouche (32) de la position ouverte à la position fermée.

13. Agrafeuse selon la revendication 12, où ladite encoche de verrouillage (110, 210) inclut une surface formant rampe (220) pour déplacer par effet de came l'axe de verrouillage.

14. Agrafeuse selon l'une des revendications précédentes, et incluant un élément de guidage de sécurité (88) comprenant :
un manchon (89) ayant des extrémités proximale et distale et étant monté d'une manière coulissante sur ledit châssis d'enclume (12) en vue d'un mouvement longitudinal relativement à celui-ci,
un axe d'alignement ou de retenue (90) s'étendant longitudinalement monté sur ladite extrémité distale dudit manchon (89) en vue d'un déplacement entre une position d'alignement où ledit axe (90) passe à travers ledit passage traversant d'alignement de boitier de cartouche (58) et s'étendant dans ladite ouverture d'alignement (15) pour positionner lesdites rangées d'agrafes (41) relativement auxdites surfaces spécialement configurées (23) sur ledit châssis d'enclume (12) pour Permettre une formation contrôlée d'une manière plus précise des agrafes tirées et pour empêcher que le tissu s'échappe depuis entre le boîtier de cartouche (40) et les surfaces d'enclume (23) lorsque l'ensemble de cartouche (32) est amené à la position fermée, et une position de relâchement où ledit axe d'alignement (90) est espacé dudit passage traversant d'alignement (58) et de ladite ouverture d'alignement (15) pour permettre le retrait et le remplacement dudit boîtier de cartouche (40) par un boîtier de cartouche neuf, et un moyen (94) monté sur ledit élément de guidage de sécurité (88) pour empêcher d'une manière répétée et continue que ledit ensemble de cartouche (32) se déplace de ladite position ouverte à ladite position fermée à moins que ledit axe d'alignement (90) ne soit dans ladite position d'alignement.

15. Agrafeuse selon la revendication 14, où le moyen (94) pour empêcher d'une manière répétée et continue que ledit ensemble de cartouche (32) se déplace de ladite position ouverte à ladite position fermée à moins que ledit axe d'alignement (90) ne se trouve dans ladite position d'alignement comprend :
ledit châssis d'enclume (12), l'élément de transport de cartouche (35) et le plongeur (48) ayant chacun des surfaces définissant des encoches de sécurité (92) qui sont alignées lorsque ledit ensemble de cartouche (32) se trouve dans ladite position ouverte,
une porte de sécurité (95) ayant des surfaces d'épaulement de came (85) et des surfaces définissant un trou, ladite porte de sécurité (95) étant montée pour être adjacente audit châssis d'enclume (12) en vue d'un mouvement relatif à celui-ci entre une position verrouillée où la porte de sécurité (95) est en prise avec les surfaces définissant les encoches de sécurité (92) pour empêcher un mouvement relatif entre l'ensemble de cartouche (32) et le châssis d'enclume (12) lorsque l'ensemble de cartouche (32) se trouve dans ladite position ouverte, et une position déverrouillée où la porte de sécurité (95) est espacée desdites encoches de sécurité (92) pour permettre un mouvement relatif de l'ensemble de cartouche (32) et du châssis d'enclume (12) entre les positions ouverte et fermée et
ledit élément de guidage de sécurité (88) incluant des guidages (98) désactivant la porte de sécurité faisant saillie proximalement depuis l'extrémité proximale dudit manchon (89) et à travers ledit trou de porte de sécurité (87), lesdits guidages (98) étant aptes à entraîner ladite porte de sécurité (95) vers ladite position verrouillée lorsque l'axe d'alignement (90) se trouve dans une position autre que la position d'alignement et ayant des surfaces de came (99) à une extrémité proximale apte à venir en prise avec les surfaces d'épaulement de came (85) de ladite porte de sécurité (95) pour entraîner la porte de sécurité (95) de ladite position verrouillée à ladite position déverrouillée lorsque l'axe d'alignement (90) est amené dans la Position d'alignement.

16. Agrafeuse selon la revendication 15, où ladite agrafeuse inclut un moyen (94) pour empêcher que ledit axe d'alignement (90) se déplace de ladite position d'alignement vers ladite position de relâchement lorsque ledit ensemble de cartouche (32) se trouve dans ladite position fermée comprenant :
lesdits guidages (98) désactivant la porte de sécurité ayant des surfaces de came de retour (8) généralement opposées auxdites surfaces de came (85) aptes à venir en prise avec les surfaces sur ladite porte de sécurité (95) afin d'entraîner la porte de sécurité (95) de la position déverrouillée vers la position verrouillée lorsque l'axe d'alignement (90) est déplacé au loin de la position d'alignement, et
des surfaces (7) sur lesdits élément de transport de cartouche (35) et plongeur (48) aptes à venir en prise avec la porte de sécurité (95) pour empêcher que l'axe d'alignement (90) se déplace de la position d'alignement vers la position de relâchement lorsque l'ensemble de cartouche (32) se trouve dans la position fermée.
